(19) Europäisches Patentamt
European Patent Office
Office européen des brevets

(11) **EP 2 961 529 B1**

(12) **EUROPÄISCHE PATENTSCHRIFT**

(45) Veröffentlichungstag und Bekanntmachung des Hinweises auf die Patenterteilung:
**20.12.2017 Patentblatt 2017/51**

(21) Anmeldenummer: **14705105.6**

(22) Anmeldetag: **13.02.2014**

(51) Int Cl.:
*C07C 29/16* *(2006.01)*     *C07C 45/78* *(2006.01)*
*C07C 45/82* *(2006.01)*     *C07C 47/02* *(2006.01)*
*C07C 31/125* *(2006.01)*   *C07C 31/02* *(2006.01)*
*B01D 61/02* *(2006.01)*

(86) Internationale Anmeldenummer:
**PCT/EP2014/052779**

(87) Internationale Veröffentlichungsnummer:
**WO 2014/131623 (04.09.2014 Gazette 2014/36)**

(54) **OPTIMIERTE TRENNTECHNIK ZUR AUFARBEITUNG VON HOMOGEN KATALYSIERTEN HYDROFORMYLIERUNGSMISCHUNGEN**

OPTIMISED SEPARATION TECHNIQUE FOR WORK-UP OF HOMOGENEOUSLY CATALYSED HYDROFORMYLATION MIXTURES

TECHNIQUE DE SÉPARATION OPTIMISÉE POUR LA RÉGÉNÉRATION DE MÉLANGES D'HYDROFORMYLATION À CATALYSE HOMOGÈNE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **26.02.2013 DE 102013203117**

(43) Veröffentlichungstag der Anmeldung:
**06.01.2016 Patentblatt 2016/01**

(73) Patentinhaber: **Evonik Degussa GmbH**
**45128 Essen (DE)**

(72) Erfinder:
• LUEKEN, Hans-Gerd
  45770 Marl (DE)
• HAMERS, Bart
  NL-5961 VG Horst (NL)
• FRIDAG, Dirk
  45721 Haltern am See (DE)
• FRANKE, Robert
  45772 Marl (DE)
• PRISKE, Markus
  Mobile, AL 36609 (US)
• HESS, Dieter
  45770 Marl (DE)

• BECKER, Marc
  44359 Dortmund (DE)
• RUDEK, Markus
  53123 Bonn (DE)

(56) Entgegenhaltungen:
EP-A1- 1 674 441       EP-B1- 1 931 472
WO-A1-2010/097428     DE-A1-102008 007 080

• YU HUANG ET AL: "Low-Energy Distillation-Membrane separation Process", INDUSTRIAL & ENGINEERING CHEMISTRY RESEARCH, AMERICAN CHEMICAL SOCIETY, US, Bd. 49, Nr. 8, 1. Januar 2010 (2010-01-01) , Seiten 3760-3768, XP002657719, ISSN: 0888-5885, DOI: 10.1021/IE901545R [gefunden am 2010-01-03]
• PRISKE M ET AL: "Reaction integrated separation of homogenous catalysts in the hydroformylation of higher olefins by means of organophilic nanofiltration", JOURNAL OF MEMBRANE SCIENCE, ELSEVIER SCIENTIFIC PUBL.COMPANY. AMSTERDAM, NL, Bd. 360, Nr. 1-2, 15. September 2010 (2010-09-15), Seiten 77-83, XP027118372, ISSN: 0376-7388 [gefunden am 2010-07-02]

**Beschreibung**

Die Erfindung betrifft ein Verfahren zur Herstellung von Alkoholen durch homogen katalysierte Hydroformylierung von Olefinen zu Aldehyden und anschließender Hydrierung der Aldehyde. Des Weiteren betrifft sie eine Anlage für die Durchführung des Verfahrens. Dabei liegt das Hauptaugenmerk der Erfindung auf der Trenntechnik zur Aufarbeitung der Hydroformylierungsmischung.

Die Hydroformylierung - auch Oxo-Reaktion genannt - ermöglicht es, Olefine (Alkene) mit Synthesegas (Gemisch aus Kohlenmonoxid und Wasserstoff) in Aldehyde umzusetzen. Die erhaltenen Aldehyde weisen dann entsprechend ein Kohlenstoff-Atom mehr auf als die eingesetzten Olefine. Durch anschließende Hydrierung der Aldehyde entstehen Alkohole, die aufgrund ihrer Genese auch "Oxo-Alkohole" genannt werden.

Grundsätzlich sind alle Olefine der Hydroformylierung zugänglich, in der Praxis werden jedoch meist solche Olefine als Substrat in der Hydroformylierung eingesetzt, die zwei bis 20 Kohlenstoffatome aufweisen. Da die durch Hydroformylierung und Hydrierung erhältlichen Alkohole vielfältig eingesetzt werden können - etwa als Weichmacher für PVC, als Detergentien in Waschmitteln und als Riechstoffe - wird die Hydroformylierung großindustriell praktiziert.

Ein Beispiel für einen weltweit stark nachgefragten Oxo-Alkohol ist Isononanol, kurz INA. Isononanol ist ein Gemisch aus isomeren Nonylalkoholen wie zum Beispiel n-Nonanol und einfach und/oder mehrfach verzweigten Nonanolen wie insbesondere Methyl-Octanol. INA trägt die CAS-Nr. 27458-94-2 und wird im Wesentlichen in der Weichmacherherstellung eingesetzt. Den $C_9$-Oxo-Alkohol INA erhält man durch Hydroformylierung von $C_8$-Olefinen wie zum Beispiel 1-Octen zu den entsprechenden $C_9$-Aldehyden und deren anschließender Hydrierung.

Wichtige Kriterien zur Unterscheidung von technischen Hydroformylierungsverfahren sind neben dem eingesetzten Substrat das Katalysatorsystem, die Phasenaufteilung im Reaktor und die Technik zum Austrag der Reaktionsprodukte aus dem Reaktor. Ein weiterer technisch relevanter Aspekt ist die Anzahl der durchgeführten Reaktionsstufen.

Industriell kommen entweder Cobalt- oder Rhodium-basierte Katalysatorsysteme zum Einsatz, wobei letztere mit Organo-Phosphor-Liganden wie Phosphin-, Phosphit- oder Phosphoramidit-Verbindungen komplexiert werden. Diese Katalysatorsysteme liegen homogen gelöst in der Reaktionsmischung vor.

Die Hydroformylierungsreaktion wird meist zweiphasig durchgeführt, mit einer flüssigen Phase, welche die Olefine, den gelösten Katalysator und die Produkte enthält sowie eine Gasphase, welche im Wesentlichen von Synthesegas gebildet wird. Die Wertprodukte werden dann entweder flüssig aus dem Reaktor abgezogen ("liquid recycle") oder gasförmig mit dem Synthesegas ausgetragen ("gas recycle"). Ein Sonderfall stellt das Ruhrchemie/Rhöne-Poulenc-Verfahren dar, bei dem der Katalysator sich in einer wässrigen Phase befindet.

Einige Hydroformylierungsverfahren werden auch in Gegenwart eines Lösungsmittels durchgeführt wie beispielsweise Alkane, die in dem Einsatzgemisch enthalten sind.

Die vorliegende Erfindung betrifft solche homogen katalysierten Hydroformylierungsverfahren, bei denen der Reaktoraustrag flüssig abgezogen wird (liquid recycle).

Da sich die Erfindung im Wesentlichen mit der Technik der Aufarbeitung des Reaktionsaustrags befasst, wird hinsichtlich der Chemie und Reaktionstechnik der Hydroformylierung auf den umfangreichen Stand der Technik verwiesen. Besonders lesenswert sind:

Falbe, Jürgen: New Syntheses with Carbon Monoxide. Springer, 1980. (Standard-Werk zur Hydroformylierung)

Pruett, Roy L.: Hydroformylation. Advances in Organometallic Chemistry. Vol. 17 Seiten 1 bis 60, 1979 (Übersichtsartikel)

Frohning, Carl D. und Kohlpaintner, Christian W.: Hydroformylation (Oxo Synthesis, Roelen Reaction). Applied homogeneous catalysis with organometallic compounds. Wiley, 1996. Seiten 29 bis 104. (Übersichtsartikel)

Van Leeuwen, Piet W.N.M und Claver, Carmen (Edit.): Rhodium Catalyzed Hydroformylation. Catalysis by Metal Complexes. Volume 22. Kluwer, 2000. (Monographie zur Rh-katalysierten Hydroformylierung. Schwerpunkt auf der Chemie, aber auch verfahrenstechnische Aspekte werden diskutiert.)

In der Patentliteratur finden sich detaillierte Verfahrensbeschreibungen zur Herstellung von INA: So offenbaren DE102008007080A1 und EP2220017B1 Co-basierte Verfahren zur Herstellung von INA. Aus EP1674441 B1 ist ein zweistufiges INA-Verfahren bekannt, bei dem auf eine Co-katalysierte Hydroformylierung eine Rh-katalysierte Oxoreaktion folgt.

Insbesondere die Abtrennung von Rh-basierten Katalysatorkomplexen aus homogen katalysierten Hydroformylierungsmischungen erweist sich als technisch anspruchsvoll. Dies liegt zum einen daran, dass Rh ein sehr teures Edelmetall ist, dessen Verlust es tunlichst zu vermeiden gilt. Aus diesem Grunde muss das Rhodium aus dem Produktstrom möglichst vollständig abgetrennt und zurückgewonnen werden. Da die Rh-Konzentration in typischen Hydroformylierungsreaktionen lediglich 20 bis 100 ppm beträgt und eine typische "world scale" Oxo-Anlage eine Jahresleistung von 200 000 Tonnen erzielt, müssen Trennapparate eingesetzt werden, die einerseits einen großen Durchsatz erlauben und andererseits das nur in geringen Mengen enthaltene Rh sicher abscheiden. Erschwerend kommt hinzu, dass die zum Katalysator-Komplex gehörigen Organo-Phosphor-Liganden sehr empfindlich auf Zustandsänderungen reagieren und rasch desaktivieren. Ein desaktivierter Katalysator ist bestenfalls nur aufwändig zu reaktivieren. Die Katalysatorabscheidung muss deshalb besonders schonend erfolgen. Ein wichtiges weiteres Entwicklungsziel ist die Energie-Effizienz der Trennoperationen.

Der Verfahrenstechniker versteht unter einer Trennoperation eine Maßnahme, bei der ein Stoffgemisch enthaltend mehrere Komponenten in mindestens zwei Stoffgemische überführt wird, wobei die erhaltenen Stoffgemische eine andere mengenmäßige Zusammensetzung als das Ausgangsgemisch aufweisen. Die erhaltenen Stoffgemische weisen in der Regel eine besonders hohe Konzentration der gewünschten Komponente auf, bestenfalls handelt es sich um Reinprodukte. Der Aufreinigungsgrad bzw. die Trennschärfe steht meist im Zielkonflikt mit der Durchsatzleistung und dem erforderlichen apparativen Aufwand und dem Energieeinsatz.

Trennverfahren lassen sich unterscheiden nach dem für die Trennung genutzten physikalischen Effekt. In der Aufarbeitung von Hydroformylierungsmischungen sind im Wesentlichen drei Gruppen von Trennverfahren bekannt nämlich thermische Trennverfahren, adsorptive Trennverfahren und Membrantrennverfahren.

Zu den thermischen Trennverfahren zählen Destillationen und Rektifikationen. Diese großindustriell bewährten Trennverfahren machen sich die unterschiedlichen Siedepunkte der im Gemisch enthaltenden Komponenten zu Nutze, indem das Gemisch verdampft und die verdampfenden Komponenten selektiv auskondensiert werden. Insbesondere hohe Temperatur und niedrige Drücke in Destillationskolonnen führen zu einer Desaktivierung des Katalysators. Aus diesem Grunde hat es bereits Ansätze gegeben, die thermische Aufarbeitung von Hydroformylierungsgemischen besonders schonend zu gestalten:

So beschreibt EP1193239B1 eine Rh-katalysierte Hydroformylierung, bei der die Produktabtrennung über einen Dünnschichtverdampfer und/oder einen Fallfilmverdampfer erfolgt. Der Katalysator wird im Verdampfer durch Aufrechterhaltung eines bestimmten Kohlenmonoxid-Partialdampfdruckes stabilisiert. Dünnschichtverdampfer und Fallfilmverdampfer sind besondere Bauarten von Apparaten zur Durchführung thermischer Trennoperationen.

Nachteil der thermischen Trennverfahren ist stets der erforderliche große Energieeinsatz. Deutlich energieeffizienter sind Membrantrennverfahren: Hierbei wird das Ausgangsgemisch als Feed auf eine Membran gegeben, welche für die unterschiedlichen Komponenten eine unterschiedliche Durchlässigkeit aufweist. Komponenten, welche die Membran besonders gut durchschreiten, werden als Permeat jenseits der Membran gesammelt und abgeführt. Komponenten, welche die Membran bevorzugt zurückhält, werden diesseits als Retentat gesammelt und abgeführt. In der Membrantechnik kommen unterschiedliche Trenneffekte zu tragen; nicht nur Größenunterschiede der Komponenten (mechanischer Siebeffekt) sondern auch Lösungs- und Diffusionseffekte werden genutzt. Je dichter die trennaktive Schicht der Membran wird, desto dominierender werden Lösungs- und Diffusionseffekte. Eine hervorragende Einführung in die Membrantechnologie bietet:

Melin / Rautenbach: Membranverfahren. Grundlagen der Modul- und Anlagenauslegung. Springer, Berlin Heidelberg 2004.

Über die Möglichkeiten des Einsatzes der Membrantechnologie zur Aufarbeitung von Hydroformylierungsgemischen berichten

Priske, M. et al.: Reaction integrated separation of homogeneous catalysts in the hydroformylation of higher olefins by means of organophilic nanofiltration. Journal of Membrane Science, Volume 360, Issues 1-2, 15 September 2010, Pages 77-83; doi:10.1016/j.memsci.2010.05.002.

Ein großer Vorteil der Membrantrennverfahren im Vergleich zu thermischen Trennverfahren ist der geringere Energieeinsatz; allerdings stellt sich auch bei Membrantrennverfahren das Problem der Desaktivierung des Katalysatorkomplexes. Gelöst wurde dieses Problem durch das in EP1931472B1 beschriebene Verfahren zur Aufarbeitung von Hydroformylierungsgemischen, bei dem sowohl im Feed, im Permeat und auch im Retentat der Membran ein bestimmter Kohlenmonoxid-Partialdampfdruck aufrecht erhalten wird. Dadurch gelingt es erstmals, die Membrantechnologie wirksam in der industriellen Hydroformylierung einzusetzen. Bei einer in Figur 3 der EP1931472B1 gezeigten, besonderen Ausführungsform sind zwei Membrantrenneinheiten mit einem Dünnschichtverdampfer kombiniert. Von dieser Ausführungsform geht die Erfindung als nächstliegenden Stand der Technik aus.

Ein spezifischer Nachteil von Membrantrennverfahren ist, dass diese noch vergleichsweise junge Technologie mit der Verfügbarkeit der Membranen steht und fällt. Spezielle Membranmaterialien, die sich für die Abscheidung von Katalysatorkomplexen eignen, sind noch nicht in großen Mengen verfügbar. Die Auftrennung großer Stoffströme erfordert aber sehr große Membranflächen und damit verbunden entsprechend viel Material und hohe Investitionskosten.

Die dritte Gruppe von Trennverfahren, die in der Aufreinigung von Hydroformylierungsmischungen genutzt werden, sind adsorptive Trennverfahren. Hier wird der Effekt der chemischen oder physikalischen Adsorption von Stoffen aus Fluiden an einem anderen flüssigen oder festen Stoff, dem Adsorbens, genutzt. Hierzu wird das Adsorbens in ein Gefäß eingefüllt und von dem zu trennenden Gemisch durchströmt. Die mit dem Fluid geführten Zielstoffe interagieren mit dem Adsorbens und bleiben so an ihm haften, sodass der aus dem Adsorber austretende Stoffstrom um die adsorbierten Stoffe abgereichert (gereinigt) ist. Technisch werden mit Adsorbentien gefüllte Gefäße auch als Scavenger bezeichnet. Man unterscheidet reversible und irreversible Adsorber, je nachdem, ob der Adsorber in der Lage ist, das adsorbierte Material wieder abzugeben (Regeneration) oder es unlösbar bindet. Da Adsorber in der Lage sind, kleinste Festkörpermengen aus Stoffströmen aufzunehmen, eignen sich adsorptive Trennverfahren insbesondere zur Feinreinigung. Für die Grobreinigung sind sie indes nicht geeignet, da das ständige Austauschen irreversibler Adsorber bzw. das ständige Regenerieren reversibler Adsorber technisch aufwändig ist.

Da sich adsorptive Trennverfahren besonders zur Abtrennung von Feststoffen eignen, sind sie dafür prädestiniert, Katalysatorrückstände aus Reaktionsmischungen abzuscheiden. Als Adsorbentien eignen sich hochporöse Materialien wie beispielsweise Aktivkohle oder funktionalisiertes Silica.

WO2009049911A1 beschreibt Adsorbentien zur Entfernung von Rhodium aus Reaktionsmischungen. Das Material basiert auf Polysiloxanen, modifiziert mit Alkylharnstoff- bzw. Alkylthioharnstoff-artigen Gruppen.

WO2006013060A1 offenbart mit Alkyl-(Thio) Ether-Gruppen modifizierte Polysiloxane, die sich ebenfalls als Adsorber zur Entfernung von Rhodium aus Reaktionsmischungen eignen sollen.

In WO2010097428A1 wird die Abtrennung von katalytisch wirksamen Rh-Komplexen aus Hydroformylierungs-Reaktionen dadurch bewerkstelligt, dass die Reaktionsmischung zunächst auf eine Membrantrenneinheit geleitet und das bereits um Rh abgereicherte Permeat sodann einem Adsorptionsschritt zugeführt wird.

Die in der diskutierten Patentliteratur beschriebenen Technologien sind grundsätzlich für Aufbereitung von Hydroformylierungsmischungen geeignet. Die jeweiligen Trenntechniken weisen - wie überall in der Technik üblich - ihre spezifischen Vor- und Nachteile auf.

Im Lichte dieses Standes der Technik besteht die Aufgabe der Erfindung darin, ein Verfahren zur Aufarbeitung von homogen katalysierten Hydroformylierungsmischungen anzugeben, welches die spezifischen Vorteile der einzelnen Trenntechnologien nutzt, dabei aber die spezifischen Nachteile weitestgehend vermeidet. Wichtigstes Ziel ist es dabei, eine möglichst vollständige und zugleich schonende Katalysatorabscheidung zu schaffen, die technisch zuverlässig arbeitet und geringe Investitions- und Betriebskosten verursacht. Das Verfahren soll uneingeschränkt tauglich sein, den Reaktionsaustrag von Oxo-Anlagen im "world scale"-Format zu verarbeiten. Gelöst wird diese Aufgabe durch geschickte Kombination unterschiedlicher Trenntechnologien unter Einhaltung bestimmter Betriebsparameter. Die erforderlichen Maßnahmen sind in Anspruch 1 niedergelegt.

Gegenstand der Erfindung ist mithin ein Verfahren zur Herstellung von Alkoholen durch homogen katalysierte Hydroformylierung von Olefinen zu Aldehyden und anschließender Hydrierung der Aldehyde mit den folgenden Schritten:

a) Bereitstellen von mindestens einem Olefin, von Synthesegas und von einem Katalysatorsystem, sowie optional von einem Lösungsmittel;

b) Hydroformylieren des Olefins in Gegenwart des Synthesegases und des Katalysatorsystems in mindestens einem Hydroformylierungsreaktor unter Bildung von zumindest Aldehyd sowie von Hochsiedern;

c) Abziehen eines flüssigen Hydroformylierungsaustrags umfassend Aldehyd, Olefin, gelöstes Synthesegas, Katalysatorsystem und Hochsieder aus dem Hydroformylierungsreaktor;

d) optionales Entgasen des flüssigen Hydroformylierungsaustrags;

e) Auftrennen des flüssigen Hydroformylierungsaustrags in einer ersten Membrantrenneinheit in einen Produktstrom und in einen Reaktorrücklauf, wobei das Katalysatorsystem im Reaktorrücklauf angereichert wird;

f) Rückführen des Reaktorrücklaufs in den Hydroformylierungsreaktor;

g) optionales Entgasen des Produktstroms;

h) Auftrennen des Produktstroms in einer thermischen Trenneinheit in ein gasförmiges Kopfprodukt umfassend Aldehyd und Olefin und in ein flüssiges Sumpfprodukt umfassend Aldehyd, Hochsieder und Katalysatorkomplex;

4

i) Auftrennen des Sumpfprodukts in einer zweiten Membrantrenneinheit in ein Permeat und in ein Retentat, wobei das Katalysatorsystem im Retentat angereichert wird;

k) bei welchem die thermische Trenneinheit dergestalt betrieben wird, dass 80% bis 98% der mit dem Produktstrom in die thermische Trenneinheit eingebrachten Masse als Kopfprodukt die thermische Trenneinheit wieder verlässt;

l) und bei welchem sowohl zumindest ein Teil des Kopfprodukts der thermischen Trenneinheit, als auch das Permeat der zweiten Membrantrenneinheit einer gemeinsamen oder getrennten Hydrierung zugeführt werden.

Eine Grundidee der vorliegenden Erfindung besteht darin, die der ersten Membrantrenneinheit nachgeschaltete thermische Trenneinheit bei vergleichsweise milden Bedingungen zu fahren, was sich darin äußert, dass 80 bis 98 % der in die thermische Trenneinheit eingebrachte Masse diese als Kopfprodukt wieder verlässt. Bevorzugt wird etwa 90% der eingetragenen Masse als Kopfprodukt abgezogen. Die Trennleistung dieser Stufe bleibt dann unter ihren technischen Möglichkeiten weit zurück, erfordert aber deutlich geringeren Energieeinsatz. Verglichen mit dem herkömmlichen Betrieb einer thermischen Trenneinheit folgt aus den erfindungsgemäß mild gewählten Betriebsbedingungen, dass das Zielprodukt (der Aldehyd) nicht vollständig über Kopf genommen wird, sondern Restaldehyd in nennenswerten Mengen im Sumpf verbleibt. Die milden Bedingungen in der thermischen Trenneinheit bewirken aber eine verringerte Desaktivierung an Katalysatorkomplex, der die erste Membrantrenneinheit überwunden hat.

Die erste Membrantrenneinheit wird nämlich ebenfalls nicht auf die vollständige Abscheidung des Katalysatorkomplexes getrimmt, was mit einer Verringerung der Membranfläche einhergeht. Bevorzugt beträgt der Rückhalt der ersten Membrantrenneinheit hinsichtlich des Katalysatorkomplexes 60 bis 90 %, bevorzugt 70 bis 80% und ganz besonders bevorzugt 75%.

Der Rückhalt R einer Membran ist über die örtlichen Konzentrationen einer Komponente i des nicht permeierenden Stroms (Retentat) sowie des durch die Membran permeierenden Stroms (Permeat) bestimmt. Sind Retentat und Permeat entlang der Membran ideal durchmischt, so entsprechen die örtlichen Retentat- und Permeatkonzentrationen den jeweiligen Konzentration des in Summe anfallenden Retentats bzw. Permeats. Der Rückhalt R einer Membran für eine im zugeführten Stoffstrom enthaltene Komponente i ist wie folgt definiert:

$$R = 1 - c_{Pi}/c_{Ri}$$

Dabei ist $c_{Pi}$ die Konzentration der Komponente i im Permeat P und $c_{Ri}$ die Konzentration der Komponente i im Retentat R. Im Grenzfall eines vollständigen Rückhalts der Komponente i durch die Membran ist $c_{Pi} = 0$ und $R = 1$. Im Fall einer bevorzugten Permeation der Komponente i ist $c_{Pi} > c_{Ri}$, und $R < 0$.

Der durch die erste Membrantrenneinheit permeierte und damit nicht rezyklierte Katalysator wird im Sumpf der thermischen Trenneinheit angereichert und erst in der zweiten Membrantrenneinheit nahezu vollständig abgeschieden und als deren Retentat zurückgeführt. Da der Sumpfstrom der thermischen Trenneinheit zwischen 2 und 20%, bevorzugt etwa 10 % der gesamten in die thermische Trenneinheit eingebrachte Masse beträgt, hat die zweite Membrantrenneinheit einen deutlich geringen Stoffstrom als die erste Membrantrenneinheit zu verarbeiten und kann dementsprechend mit vertretbarem apparativem Aufwand eine nahezu vollständige Katalysatorabscheidung bewerkstelligen. Insbesondere ermöglicht der geringe Stoffstrom den wirtschaftlichen Einsatz einer zweistufigen Membrantrenneinheit, die bessere Trennergebnisse erzielt. Dazu später mehr.

Ein weiterer Vorteil der beschriebenen Betriebsweise der thermischen Trenneinheit besteht darin, dass sich die im Hydroformylierungsreaktor gebildeten Hochsieder ebenfalls im Sumpf der thermischen Trenneinheit konzentrieren und als Lösungsmittel für den Katalysator dienen. Die Abscheidung des Katalysators erfolgt also in der ersten Membrantrenneinheit aus dem Austrag des Hydroformylierungsreaktors, währenddessen an der zweiten Membrantrenneinheit die Katalysatorabscheidung aus dem Hochsiederstrom erfolgt. Da beide Membranen in unterschiedlichen Stoffsystemen arbeiten, lässt sich die insgesamt erzielbare Abscheideleistung end-optimieren. Die Endoptimierung erfolgt durch geeignete Wahl des Membranmaterials, der Verschaltung innerhalb der Membrantrennstufe und der Betriebsbedingungen der einzelnen Membranen.

Die Abscheidung des Katalysators wird auch durch die Verfügbarkeit von Membranmaterialien erleichtert, welche für den Einsatz in Hochsiederströmen besonders geeignet sind. Zugleich wird eine Rückführung der Hochsieder über den am Retentat der zweiten Membrantrenneinheit beginnenden, sekundären Kreislauf in den Hydroformylierungsreaktor vermieden, was eine die Raum-Zeit-Ausbeute der Reaktion beeinträchtigende Hochsiederkonzentrierung im Reaktor verhindert.

Die Aufkonzentrierung von Hochsiedern im Hydroformylierungsreaktor kann auch dadurch vermieden werden, dass das Retentat der zweiten Membrantrenneinheit nicht in den Hydroformylierungsreaktor zurückgeführt wird, sondern mit dem aus dem Hydroformylierungsreaktor abgezogenen, flüssigen Hydroformylierungsaustrag vermischt und der ersten Membrantrenneinheit zugeführt wird. Der sekundäre Kreislauf wird somit nicht vor den Hydroformylierungsreaktor zurückgeführt, sondern dahinter.

Dies hat zunächst den Vorteil, dass selbst dann, wenn eine hinsichtlich der Hochsieder weniger selektive Membran eingesetzt wird, die Hochsieder nicht als Inertkomponenten in die Hydroformylierung geführt werden. Ein hoher Anteil von Inertkomponenten im Reaktor führt zu Ausbeuteverlusten. Ein weiterer wichtiger Vorteil der Rückführung des Retentats der zweiten Membrantrenneinheit in den Feed der ersten Membrantrenneinheit anstatt in den Reaktorzulauf besteht darin, dass sich die Regelungstechnik der Membrantrenneinheiten besser von der Regelungstechnik des Reaktors isolieren lässt und auf diese Weise gegenseitige Interferenzen reduziert werden. So nimmt ein Hydroformylierungsreaktor insbesondere bei Verarbeitung von Rohstoffen, deren stoffliche Zusammensetzung Schwankungen unterliegt, einen weniger stationären Zustand ein als Trennapparat. Dies bedeutet, dass die Regelung des Reaktors deutlich dynamischer arbeitet als die der Trennapparate. Ein Zurückführen des Retentats der zweiten Membrantrenneinheit vor den Reaktor führt zu einer weiteren Koppelung dieser Regelkreise, sodass sich instationäres Verhalten des Reaktors auch auf die Membrantrenneinheiten auswirkt. Durch Rückführen des Retentats der zweiten Membranstufe hinter den Hydroformylierungsreaktor wird dieses Problem weitestgehend vermieden. Natürlich wird dieser Vorteil auch dann genutzt, wenn die in der zweiten Membrantrenneinheit eingesetzte Membran eine hohe Durchlässigkeit für Hochsieder aufweist und somit zur Hochsieder-Ausschleusung genutzt wird.

Der Anteil an Katalysator, welcher die erste Membrantrenneinheit überwindet und damit nicht in primären Katalysatorkreislauf vor den Reaktor zurückgeführt wird, wird bei der erfindungsgemäßen Anlage in die thermische Trenneinheit eingebracht. Bei einem Rückhalt der ersten Membrantrenneinheit von 75 % gelangen also die verbleibenden 25% des Katalysator-Ligand-Komplexes in die thermische Trenneinheit. Der Katalysator-Ligand-Komplex verlässt die thermische Trenneinheit im Wesentlichen über deren Sumpfstrom. Es ist jedoch zu erwarten, das geringe Mengen an Katalysator von dem in der thermischen Trenneinheit entstehenden Dampf mitgerissen und über Kopf ausgetragen werden. Mithin werden sich im Kopfprodukt der thermischen Trenneinheit geringe Mengen an Katalysator finden.

Um zu verhindern, dass diese Restmengen mit dem im Kopfprodukt vorherrschenden Aldehyd aus der Anlage ausgetragen und damit unwiederbringlich verloren zu gehen, sieht eine bevorzugte Weiterbildung der Erfindung vor, das Kopfprodukt der thermischen Trenneinheit vor der Hydrierung durch einen Adsorber zu fahren. Der Adsorber fängt die Katalysator-Restmengen ein und hält diese zurück. Da der Großteil der Katalysatorrückstände über die erste Membrantrenneinheit bzw. über den Sumpf der thermischen Trenneinheit rezykliert werden, sind im Kopfprodukt der thermischen Trenneinheit nur noch geringe Mengen an Katalysator zu erwarten. Der Einsatz des Adsorbers ist deswegen an dieser Stelle sachdienlich.

Bei dem Adsorber handelt es sich um einen von dem kondensierten Kopfprodukt durchströmtes Gefäß, welches mit einem Adsorbens gefüllt ist, wie beispielsweise ein modifiziertes Silica oder Aktivkohle. Der Adsorber verfügt über keine bewegten Bauteile und hat auch keinen Eigenenergiebedarf, weswegen seine Betriebskosten vergleichsweise gering sind. Da nur geringe Mengen an zu adsorbierendem Katalysator erwartet werden, und der Katalysator deutlich teurer ist als das Adsorbens, ist der Einsatz eines irreversiblen Adsorbens wirtschaftlich.

Die Anordnung des Adsorbers vor der Hydrierung bewirkt den Vorteil, dass sich innerhalb des Hydrierreaktors kein Rhodium niederschlägt. So weist ein technisch ausgeführter Hydrierreaktor durchaus die Fähigkeit auf, Rhodium zu adsorbieren; jedoch setzen sich die Metalle an der Innenwand des Reaktors ab und lassen sich von dieser nur unter einem erhöhten Arbeitsaufwand wieder lösen. Die Wiedergewinnung des Rhodiums aus dem Adsorbens ist indes vielfach einfacher. Deshalb ist es sinnvoll den Katalysator vor der Hydrierung zu adsorbieren. Auch ist ein eigens eingesetzter Adsorber trennschärfer und daher selektiver als ein Hydrierreaktor.

Des Weiteren kann nicht ausgeschlossen werden, dass geringste Katalysatormengen die zweite Membrantrenneinheit überwinden. Zwar wird eine Auslegung der zweiten Membrantrenneinheit angestrebt, bei welcher der Katalysator über den sekundären Kreislauf nahezu vollständig zurückgeführt wird, jedoch erreicht auch eine Membran nie eine ideale Stofftrennung. Der Katalysator, der die zweite Membrantrenneinheit überwindet und sich in ihrem Permeat findet, sollte demnach gemäß einer bevorzugten Weiterbildung der Erfindung dadurch vor Verlust geschützt werden, dass das Permeat der zweiten Membrantrenneinheit vor der Hydrierung durch einen Adsorber gefahren wird. Bezüglich des hinter der zweiten Membrantrenneinheit angeordneten Adsorbers gilt prinzipiell das für den hinter der thermischen Trenneinheit angeordneten Adsorber Gesagte.

Die adsorptiv gereinigten Stoffströme Kopfprodukt der thermischen Trenneinheit und Permeat der zweiten Membrantrenneinheit werden getrennt oder gemeinsam einer Hydrierung unterworfen. Die Hydrierung hat primär den Zweck, die in der Hydroformylierung gebildeten Aldehyde in die entsprechenden Alkohole zu überführen. Freilich findet bereits im Oxo-Reaktor eine Hydrierung der Aldehyde als Folgereaktion der Hydroformylierung statt; bei Rh-katalysierten Oxo-Prozessen sind das allerdings nur etwa 3 %. Die Hydrierung im Hydroformylierungsreaktor ist eine bestenfalls geduldete Folgereaktion, welche nicht die erforderliche Ausbeute an Alkoholen bewirkt. Die Haupt-Hydrierleistung wird von dem Hydrierreaktor erbracht. Im Übrigen sind auch die Reaktionsbedingungen in der Hydrierung an diese Reaktion besser angepasst und entsprechende Katalysatoren zugegen. Eine weitere Funktion der Hydrierung besteht darin, die der Oxo-Reaktion entkommenen Rest-Olefine zu Alkanen umzusetzen und sie dadurch hinsichtlich ihrer Reaktivität zu entschärfen. Ein Teil der Olefine werden im Übrigen auch im Oxo-Reaktor hydriert.

Spätestens hinter der Hydrierung liegen also die erfindungsgemäß hergestellten Alkohole vor. Sie befinden sich in dem aus dem Hydrierreaktor abgezogenen Hydrierungsgemisch und werden begleitet von besagten Alkanen als Leichtsieder sowie von hydrierten Hochsiedern. Eine bevorzugte Weiterbildung der Erfindung sieht demnach vor, das aus der Hydrierung abgezogene Hydrierungsgemisch einer thermischen Aufarbeitung zu unterziehen unter Erhalt einer alkoholreichen Fraktion, einer Leichtsiederfraktion und einer Hochsiederfraktion.

Die Aufarbeitung des Hydrierungsgemischs erfolgt mittels klassischer Destillations-Technologie, da diese sich bewährt hat und auch keine Rücksicht mehr auf Katalysatorreste genommen werden muss. Die thermische Aufarbeitung erfolgt bevorzugt dreistufig mit Hilfe von dreier hintereinander geschalteten Destillationskolonnen:

In einer ersten Destillationskolonne wird die Leichtsiederfraktion über Kopf aus dem Hydrierungsgemisch abgetrennt. Im Sumpf der ersten Destillationskolonne sammeln sich die Hochsieder sowie der Alkohol. Die über Kopf abgetrennte Leichtsiederfraktion wird aus dem Prozess ausgeschleust und als Wertprodukt verkauft. Werden beispielsweise C9-Alkohole aus $C_8$-Olefinen hergestellt, umfasst die Leichtsiederfraktion überwiegend C8-Alkane sowie eine geringe Menge in der Hydrierung nicht umgesetzter C9-Aldehyd. Ein solches "leichtes Oxoöl" kann als Lösemittel für verschiedene Bereiche verwendet werden.

Das über den Sumpf der ersten Destillationskolonne ausgetragene Gemisch aus Hochsiedern und Alkoholen wird nun einer zweiten Destillationskolonne aufgegeben. Diese trennt über Kopf eine alkoholreiche Fraktion ab, die das eigentliche Hauptwertprodukt des erfindungsgemäßen Verfahrens darstellt. Im Sumpf der zweiten Destillationskolonne sammelt sich überwiegend die Hochsiederfraktion begleitet von Rest-Alkohol.

Diese Sumpffraktion wird einer dritten Destillation zugeführt, über deren Kopf der verbleibende Alkohol gewonnen wird. Im Sumpf der dritten Destillationskolonne wird die Hochsiederfraktion gewonnen und als Nebenprodukt vermarktet. Bei dem auf diese Weise erhaltenen "schweren Oxoöl" handelt es sich um eine hoch siedende Flüssigkeit mit hohem Flammpunkt, welche vorwiegend als Lösemittel in der Mineralölindustrie, als Hilfsmittel in der Lack-, Leder- und Gummiindustrie, sowie in der Papier- und Textilherstellung verwendet wird.

Je nach Fahrweise der Oxo-Reaktion ist der Umsatz der eingetragenen Alkene nicht vollständig. In der Praxis kann der Umsatz auf etwa 93 % begrenzt sein, was bedeutet, dass etwa 7 % der eingetragenen Alkene den Oxo-Reaktor wieder mit dem Reaktionsgemisch verlassen. Die nicht umgesetzten Alkene gehen spätestens in der Hydrierung verloren. Um dies zu verhindern, kann die Hydrierung auch hinter der Produktabtrennung angeordnet werden was zur Folge hat, dass die sich in der Leichtsiederfraktion auch die nicht umgesetzten Alkene finden. Diese können dann zurück in den Oxo-Reaktor geführt werden, um dort erneut der Hydroformylierung vorgelegt zu werden. Auf diese Weise wird der Verlust von Alkenen verringert. Falls also die Hydrierung hinter der Produktabtrennung angeordnet wird, wird das Kopfprodukt der thermischen Trenneinheit nicht vollständig, sondern nur teilweise der Hydrierung zugeführt; die darin enthaltenen Alkene werden in die Oxo-Reaktion rezykliert. Nachteil dieser Ausführungsform ist, dass für jede der erhaltenen Fraktionen eine eigene Hydrierung vorgesehen werden muss.

Nach diesem Exkurs zur Aufarbeitung des Hydrierungsgemisches und zur Anordnung der Hydrierung zurück zu der vornehmlich interessierenden Aufarbeitung des Hydroformylierungsaustrags:

Die thermische Trenneinheit wird einfachstenfalls mit einer Destillationskolonne realisiert. Eine bevorzugte Ausführungsform der Erfindung sieht jedoch vor, dass die thermische Trenneinheit aus einem Fallfilmverdampfer und einem Dünnschichtverdampfer gebildet wird, wobei Dünnschichtverdampfer und Fallfilmverdampfer seriell miteinander verschaltet sind. Dabei wird der Dünnschichtverdampfer bevorzugt hinter dem Fallfilmverdampfer angeordnet. Dünnschichtverdampfer und Fallfilmverdampfer sind an sich bekannte thermische Trennapparate.

Der Dünnschichtverdampfer (engl.: thin film evaporator) weist eine im Wesentlichen zylindrische, mit Dampf beheizte Innenwandung auf, auf der mittels rotierender Verteilorgane eine dünne Schicht des Ausgangsgemischs aufgebracht wird. Die motorisch angetriebenen Verteilorgane ("Wischer") werden benötigt, um das rasch auf den Platten verdampfende Gemisch auszubringen und zu verteilen.

Der Fallfilmverdampfer (engl.: falling film evaporator) umfasst ein im Wesentlichen vertikal verlaufendes, von außen beheiztes Rohr, an dessen Innenseite das Ausgangsgemisch in einem dünnen Film herab rinnt und dabei verdampft. Die unverdampften Komponenten werden am bodenseitigen Ende des Rohrs als Sumpf abgezogen, die verdampften Komponenten verlassen das andere Ende des Rohrs als Kopfprodukt. Der Fallfilmverdampfer kommt mithin ohne bewegliche Teile aus.

Gemeinsames konstruktives Merkmal von Dünnschichtverdampfer und Fallfilmverdampfer ist also mindestens ein beheiztes, flächiges Verdampfungsorgan, auf dem eine dünne Schicht des flüssigen Ausgangsgemisches aufgegeben und teilweise verdampft wird. Eine eingehende Beschreibung von Dünnschichtverdampfer und Fallfilmverdampfer findet sich im ULLMANN:

Billet, Reinhard: Evaporation. Ullmann's Encyclopedia of Industrial Chemistry. Published Online: 15 JUN 2000 DOI: 10.1002/14356007.b03_03

Alternativ ist es möglich, die thermische Trenneinheit mit Hilfe von zwei oder drei in Serie geschalteten Fallfilmverdampfern zu realisieren. Dies ermöglicht eine Flächen- und Verweilzeitminimierung, erhöht die Betriebsflexibilität und führt zu einer für den Katalysator besonders schonenden Trennung.

Die Trennapparate zur Realisierung der thermischen Trenneinheit werden bevorzugt bei einem Unterdruck zwischen 3 und 500 hPa betrieben.

Die erste Membrantrenneinheit wird einfachstenfalls als "Feed-and-Bleed"-System mit einem einzelnen Rezirkulationskreislauf (einem so genannten Loop) ausgeführt. Im Rezirkulationskreislauf wird ein Teil des Retentats in den Feed zurückgeführt.

Die Permeatqualität kann zunächst dadurch verbessert werden, dass die Membrantrenneinheit aus mehreren seriellen Loops aufgebaut wird.

Weiter kann die Permeatqualität dadurch verbessert werden, dass ein mehrstufiges Feed-and-Bleed-System als Membrantrenneinheit genutzt wird. Dabei handelt es sich um eine mehrstufige Membrankaskade, welche über mehrere Rezirkulationskreisläufe bzw. Loops verfügt. Kaskadierte Feed-and-Bleed-Membransysteme können entweder als "Abtriebskaskade" oder als "Verstärkerkaskade" ausgeführt sein. Jede Stufe einer solchen Kaskade kann aus einen oder mehreren Loops aufgebaut werden.

Gegenüber einer in den Membrantechnik ebenfalls gebräuchlichen "Tannenbaumverschaltung" erlauben als "Feed und Bleed" System aufgebaute Membrankaskaden den Betrieb bei in Qualität und/oder Quantität schwankender Zulaufbedingungen und/oder sich zeitlich verändernder Membranperformance. Bei hohen Konzentrierungsfaktoren führt eine Abtriebskaskade zu einer besseren Gesamtpermeatqualität als eine Verstärkungskaskade bei gleicher verbauter Membranfläche. Weiterhin besitzt eine Verstärkungskaskade den Nachteil, dass benötigte Fläche gegenüber einer einstufigen Membrantrenneinheit mehr als doppelt so groß ist. Bei einer Abtriebskaskade hingegen können auch nahezu beliebige Membranfläche zwischen einer einstufigen Membrantrenneinheit und einer Verstärkungskaskade umgesetzt werden. Dies ist insbesondere dann wichtig, wenn eine Verstärkungskaskade aufgrund der hohen benötigten Membranfläche nicht wirtschaftlich ist und eine einstufige Membrantrenneinheit aufgrund unzureichender Abtrennung nicht einsetzbar ist.

Aus diesen Gründen bietet es sich an, eine zweistufige Membrankaskade mit teilweiser Permeatrückführung als erste Membrantrenneinheit einzusetzen. Zurückgeführt wird dabei das Permeat aus dem Rezirkulationskreislauf bzw. den Kreisläufen mit der schlechtesten Permeatqualität, welches in der Regel die Loops mit der höchsten Retentatkonzentration am Ende der Aufkonzentrierungsstrecke sind. Diese Verschaltung wird in der Membrantechnik "zweistufige Abtriebskaskade" genannt. Die Rezirkulationskreisläufe mit Permeatrückführung am Ende der Kaskade werden auch als Konzentratloops bezeichnet. Eine Verschaltung mit Konzentratloops mit Permeatrückführung ermöglicht ein reineres Gesamtpermeat.

Ganz besonders bevorzugt wird die genutzte Membranfläche der Konzentratloops kleiner ausgeführt als die genutzte Membranfläche der übrigen Loops. Auf diese Weise wird ohne Verschlechterung des Trennergebnisses der Membranflächenbedarf gesenkt.

Die zweite Membrantrenneinheit wird bevorzugt als zweistufige Verstärkungskaskade ausgeführt. Bei einer zweistufigen Verstärkungskaskade handelt es sich um eine mehrstufige Membrankaskade mit teilweiser Retentatrückführung. Zurückgeführt wird dabei das Gesamtretentat aus der zweiten Stufe. Sowohl die erste als auch die zweite Stufe der zweistufigen Verstärkungskaskade können aus einem oder mehreren Membranloops aufgebaut werden. Zum einen sind die hier benötigten Konzentrierungsfaktoren niedrig genug, dass eine Verstärkungskaskade aufgrund des Trennergebnisses gegenüber einer Abtriebskaskade vorteilhaft ist. Zum anderen sind die zu erzeugenden Permeatmengen und damit die benötigen Membranflächen so klein, dass eine Verstärkungskaskade wirtschaftlich ist.

Die erfindungsgemäße Aufarbeitung von Hydroformylierungsmischungen ist grundsätzlich auf jedwede homogene katalysierte Hydroformylierung mit flüssigem Reaktionsaustrag (liquid recycle) anwendbar. Bevorzugt wird sie jedoch auf Rhodiumkatalysierte Hydroformylierungen angewendet, da diese Katalysatorsysteme aufgrund des hohen Rhodium-Preises eine besonders sorgfältige Katalysatorabtrennung erfordern. Ganz besonders bevorzugt wird die Erfindung bei Prozessen angewandt, in denen ein Rhodium-System als Katalysator eingesetzt wird, welches einen Organo-Phosphor-Liganden besitzt. Zu den Organo-Phosphor-Liganden zählen Phosphine, Phosphite und Phosphoramidite. Besonders interessant ist der Einsatz von RhodiumKatalysatoren mit Phosphit-Liganden, da diese durch ihre besonders hohe Selektivität bestechen. Allerdings sind die Phosphite besonders anfällig gegen Zersetzung. Da das erfindungsgemäße Verfahren auf eine besonders schonende Katalysatorabtrennung ausgerichtet ist, ermöglicht es den wirtschaftlichen Einsatz von Rhodium-Phosphit-Systemen im industriellen Maßstab.

Da die Chemie und Reaktionstechnik der Rhodium-katalysierten Hydroformylierung ausführlich in

Van Leeuwen, Piet W.N.M und Claver, Carmen (Edict.): Rhodium Catalyzed Hydroformylation. Catalysis by Metal Complexes. Volume 22. Kluwer, 2000.

beschrieben wird, erübrigen sich weitergehende Ausführungen dazu.

In dem erfindungsgemäßen Verfahren können grundsätzlich alle hydroformylierbaren Olefine eingesetzt werden. Dies sind in der Regel solche Olefine mit 2 bis 20 Kohlenstoffatomen. In Abhängigkeit von dem eingesetzten Katalysatorsystem können sowohl endständige, als auch nicht-endständige Olefine hydroformyliert werden. Rhodium-Phosphit-Systeme können sowohl endständige, als auch nicht-endständige Olefine als Substrat verwenden.

Die eingesetzten Olefine müssen auch nicht als Reinstoff eingesetzt werden, vielmehr können auch Olefingemische als Edukt genutzt werden. Olefingemische sind zu verstehen einerseits als Gemische verschiedener Isomere von Olefinen mit einer einzigen Anzahl von Kohlenstoffatome; andererseits kann ein Olefingemisch aber auch Olefine mit unterschiedlichen Anzahlen von Kohlenstoffatomen und deren Isomere umfassen. Ganz besonders bevorzugt werden Olefine mit 8 Kohlenstoffatomen in dem Verfahren eingesetzt und mithin zu Aldehyden mit 9 Kohlenstoffatomen hydroformyliert und anschließend die Aldehyde zu Alkohol mit 9 Kohlenstoffatomen hydriert. Auf diese Weise erhält man aus den $C_8$-Olefinen Isononanol (INA).

Das erfindungsgemäße Verfahren wird ausgeführt und verkörpert in einer entsprechenden Anlage für die Herstellung von Alkohol. Eine solche Anlage ist hier ebenfalls offenbart und weist die folgenden Apparate auf:

a) mindestens einen Hydroformylierungsreaktor mit einem Edukteinlass und einem Produktauslass,

b) eine erste Membrantrenneinheit mit einem ersten Membraneingang, einem ersten Permeatanschluss und einem ersten Retentatanschluss,

c) eine thermische Trenneinheit mit einem Produkteinlass, einem Kopfanschluss und einem Sumpfanschluss,

d) eine zweite Membrantrenneinheit mit einem zweiten Membraneingang, einem zweiten Permeatanschluss und einem zweiten Retentatanschluss,

e) mindestens einen Hydrierreaktor mit einem Aldehydeingang und einem Alkoholausgang.

Diese Apparate sind Stoff übertragend wie folgt miteinander verschaltet:

f) der Produktauslass des Hydroformylierungsreaktors ist direkt oder über einen Entgaser auf den ersten Membraneingang der ersten Membrantrenneinheit geschaltet,

g) der erste Retentatanschluss der ersten Membrantrenneinheit ist auf den Edukteinlass des Hydroformylierungsreaktors geschaltet,

h) der erste Permeatanschluss der ersten Membrantrenneinheit ist direkt oder über einen Entgaser auf den Produkteinlass der thermischen Trenneinheit geschaltet,

i) der Sumpfanschluss der thermischen Trenneinheit ist auf den zweiten Membraneingang der zweiten Membrantrenneinheit geschaltet,

k) der Kopfanschluss der thermischen Trenneinheit ist direkt oder über einen Adsorber auf den Aldehydeingang des Hydrierungsreaktors geschaltet,

l) der zweite Retentatanschluss der zweiten Membrantrenneinheit ist zusammen mit dem Produktauslass des Hydroformylierungsreaktors auf den ersten Eingang der ersten Membrantrenneinheit geschaltet,

m) der zweite Permeatanschluss der zweiten Membrantrenneinheit ist direkt oder über den Adsorber auf den Aldehydeingang des Hydrierungsreaktors geschaltet.

Die Besonderheit der Anlage besteht darin, dass die sekundäre Katalysatorrückführung ausgehend von dem zweiten Retentat am Schluss der zweiten Membrantrenneinheit auf den Eingang der ersten Membrantrenneinheit eingeschaltet ist und nicht auf den Eingang des Hydroformylierungsreaktors. Dies verhindert Hochsiederbildung im Hydroformylierungsreaktor und verbessert insbesondere die Regelbarkeit der erfindungsgemäßen Anlage.

Eine weitere Schaltungsbesonderheit dieser Anlage besteht darin, dass beide vom Katalysator befreiten Produktströme vom Kopf der thermischen Trenneinheit und aus dem Permeat der zweiten Membrantrenneinheit auf den Aldehydeingang des Hydrierungsreaktors geschaltet sind. Des Weiteren ist die Verwendung dieser Anlage zur Durchführung eines erfindungsgemäßen Verfahrens offenbart. Weitere bevorzugte Ausführungsformen der Erfindung ergeben sich aus der nun folgenden detaillierten Beschreibung einer Anlage und des damit durchgeführten erfindungsgemäßen Verfahrens. Hierfür zeigen:

Figur 1: Schaltbild einer ersten Ausführungsform mit zwei getrennten Adsorbern;
Figur 2: Schaltbild einer zweiten Ausführungsform einer Anlage mit einem gemeinsamen Adsorber;
Figur 3: Detaildarstellung der Aufreinigung;
Figur 4: Erste Ausführungsform der thermischen Trenneinheit bestehend aus Fallfilmverdampfer und Dünnschichtverdampfer;
Figur 5: Zweite Ausführungsform der thermischen Trenneinheit bestehend aus zwei Fallfilmverdampfern;
Figur 6: Detaildarstellung erste Membrantrenneinheit als zweistufige Abtriebskaskade;
Figur 7: Detaildarstellung zweite Membrantrenneinheit als zweistufige Verstärkungskaskade.

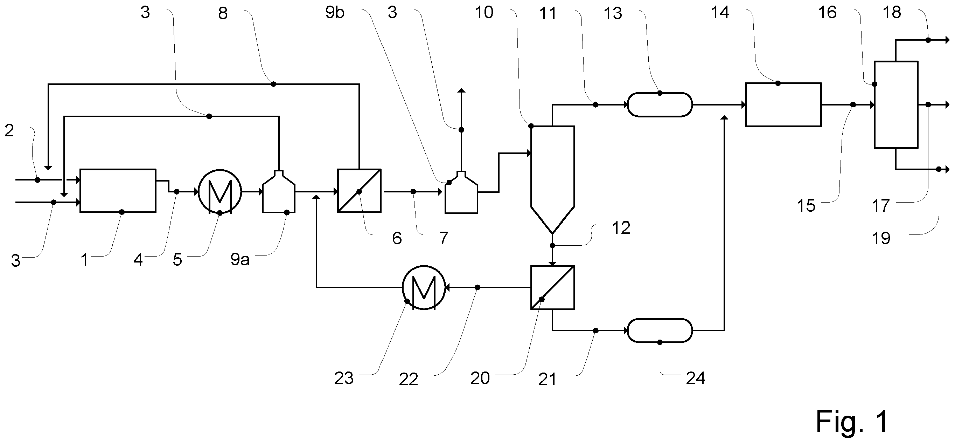

Figur 1 zeigt das Schaltbild einer Anlage mit welcher das erfindungsgemäße Verfahren durchgeführt werden kann. Wie üblich ist das Schaltbild vereinfacht, um die Lesbarkeit zu verbessern. Selbstverständliche Anlagenkomponenten wie Ventile, Pumpen und dergleichen sind nicht dargestellt.

Kernstück der Anlage bildet ein Hydroformylierungsreaktor 1. Hierin findet die Hydroformylierungsreaktion statt. Dabei wird ein Olefin 2 mit Synthesegas 3 - eine Mischung aus Kohlenmonoxid und Wasserstoff - in Gegenwart eines homogen gelösten Katalysators zu entsprechenden Aldehyden mit einem Kohlenstoffatom mehr umgesetzt. Bei dieser Reaktion handelt es sich um eine Gas/Flüssigphasen-Reaktion, bei welcher das Olefin und die Reaktionsprodukte in der flüssigen Phase vorliegen, währenddessen ein Teil des Synthesegases 3 die Gasphase bildet und ein anderer Teil des Synthesegases in der flüssigen Phase gelöst ist. Ebenfalls in der flüssigen Phase gelöst ist ein homogener Katalysatorkomplex.

Optional kann dem Hydroformylierungsreaktor 1 ein Lösungsmittel zugeführt werden, etwa Alkane, die das eingesetzte Olefin begleiten. Die Hydroformylierung findet dann in Gegenwart des optionalen Lösungsmittels statt.

Als Reaktorbauart kommen grundsätzlich solche Apparate in Betracht, die eine Gas-Flüssigphasenreaktion erlauben. Bevorzugt wird ein Blasensäulen-Reaktor eingesetzt. Blasensäulen-Reaktoren sind im Stand der Technik allgemein bekannt und werden ausführlich im ULLMANN beschrieben:

Deen, N.G., Mudde, R.F., Kuipers, J.A.M., Zehner, P. and Kraume, M.: Bubble Columns. Ullmann's Encyclopedia of Industrial Chemistry. Published Online: 15 January 2010. DOI: 10.1002/14356007.b04_275.pub2

Da Blasensäulenreaktoren aufgrund ihres Strömungsverhaltens sich nicht beliebig skalieren lassen, ist es bei einer Anlage mit sehr großer Produktionskapazität erforderlich, statt eines einzelnen großen Reaktors zwei oder mehr parallel geschaltete kleinere Reaktoren vorzusehen. So können bei einer world-scale Anlage mit einer Leistung von 30 t/h entweder zwei oder drei Blasensäulen mit jeweils 15 t/h bzw. 10 t/h Kapazität vorgesehen werden. Die Reaktoren arbeiten bei denselben Reaktionsbedingungen parallel. Die Parallelschaltung mehrerer Reaktoren hat auch den Vorteil, dass bei einer geringeren Anlagenauslastung der Reaktor nicht im energetisch ungünstigen Teillastbereich gefahren werden muss. Stattdessen wird einer der Reaktoren komplett abgeschaltet und der andere Reaktor weiterhin unter Volllast gefahren. Eine Dreifachverschaltung kann entsprechend noch flexibler auf Bedarfsänderungen reagieren.

Wenn also hier von einem Hydroformylierungsreaktor die Rede ist, heißt das nicht zwangsläufig, dass es sich nur um einen Apparat handelt. Es können auch mehrere miteinander verschaltete Reaktoren gemeint sein.

Die Reaktion wird bei üblichen Bedingungen durchgeführt.

Besonders bevorzugt sind eine Temperatur von 120 °C bis 160 °C und ein Druck von 20 bis 28 MPa. Bei diesen Bedingungen wird ein Umsatz von > 90 % angestrebt. Synthesegas mit einem Wasserstoff/Kohlenmonoxid-Verhältnis von 1:1 wird im Überschuss in den Reaktor gefahren.

Als Substrat für die Hydroformylierung kommen grundsätzlich alle Olefine in Betracht, die der Oxo-Reaktion zugänglich sind. Dies sind insbesondere die Olefine mit zwei bis zwanzig Kohlenstoffatomen. Besonders bevorzugt werden C6 - C12 Olefingemische eingesetzt. Für die INA-Produktion verwendet man Olefingemische mit einem hohen Gehalt an Isoocten wie beispielsweise das bei Evonik Industries erhältliche Di-n-Buten (CAS-Nr. 10071-47-9).

Als Katalysatorsystem kommen die folgenden Homogenkatalysatoren in Betracht:

Besonders werden Rhodium-Phosphit-Systeme als Homogenkatalysator eingesetzt. Ein solches System kann zum Beispiel aus Rhodiumnonanoat und Tris(2,4-di-tert-butylphenyl)phosphit dargestellt werden. Die Metallkonzentration sollte zwischen 5 und 100 ppm liegen und das Ligand/Rhodium-Verhältnis bevorzugt etwa 5:1 betragen.

In der Hydroformylierung bilden sich neben den gewünschten Aldehyden in anschließender Folgereaktion auch die entsprechenden Alkohole sowie Hochsieder. Zu den Hochsiedern gehören unter anderem Dimere, Trimere, Aldolprodukte, Tishchenko-Produkte, Ester und Ether. Die Hochsiederbildung in der Reaktion ist unerwünscht, da sie zu Ausbeuteverlusten führt, ist aber reaktionstechnisch nicht völlig zu vermeiden. Die Hochsieder müssen deswegen entsprechend ihrer Bildungsrate aus dem Prozess ausgeschleust werden. Die Bezeichnung Hochsieder rührt daher, dass diese Substanzen einen höheren Siedepunkt aufweisen als der Aldehyd, weswegen sich die Hochsieder am Sumpf der thermischen Trenneinheit bzw. der Destillation hinter der Hydrierung ansammeln. Demgegenüber gehören zu den Leichsiedern Olefine, Alkane und Aldehyde, die in der Hydroformylierung oder der Hydrierung gebildet werden bzw. bereits in dem Olefingemisch vorhanden sind.

Aus dem Reaktor 1 wird ein flüssiger Hydroformylierungsaustrag 4 abgezogen, welcher neben dem gewünschten Aldehyd auch unumgesetztes Olefin, in der Flüssigkeit gelöstes Synthesegas, das homogen gelöste Katalysatorsystem, weitere Leichtsieder und die Hochsieder enthält. Sofern ein optionales Lösungsmittel eingesetzt wird, gehört dieses zu den Leichtsiedern.

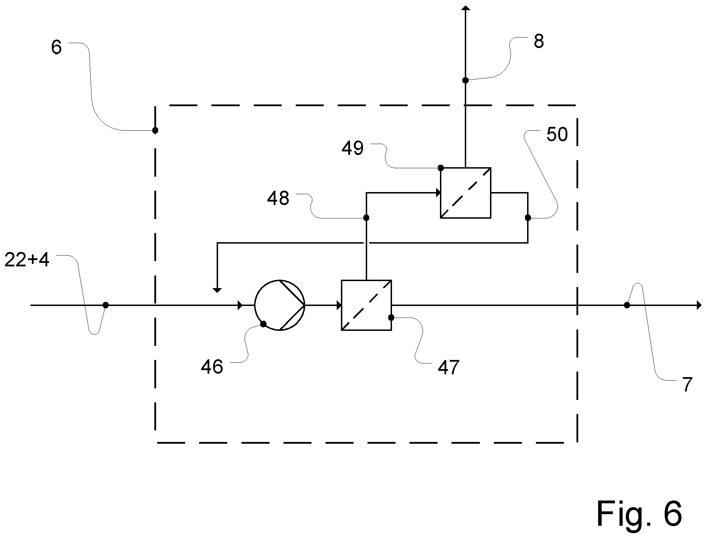

In einem ersten Wärmetauscher 5 wird der Hydroformylierungsaustrag 4 auf eine Temperatur von etwa 40 bis 50 °C abgekühlt. In einem ersten Entgaser 9a wird der Hydroformylierungsaustrag 4 auf etwa 0.5 MPa entspannt und das dabei ausperlende Synthesegas 3 in den Reaktor 1 zurückgeführt. Sodann wird der Hydroformylierungsaustrag 4 einer ersten Membrantrenneinheit 6 aufgegeben. Bei der Membrantrenneinheit 6 handelt es sich um eine mehrstufige Abtriebskaskade, die Anhand der Figur 6 näher erläutert werden wird. Für den funktionalen Zusammenhang genügt es aber, die erste Membrantrenneinheit 6 wie eine Einzelmembran aufzufassen.

Der anströmende Hydroformylierungsaustrag 4 wird in der ersten Membrantrenneinheit 6 in einem Produktstrom 7 und in einen Reaktorrücklauf 8 aufgetrennt, wobei das im Hydroformylierungsaustrag 4 enthaltene Katalysatorsystem im Reaktorrücklauf 8 angereichert wird. Der Produktstrom 7 stellt dabei das Permeat der ersten Membrantrenneinheit 6 dar, währenddessen der Reaktorrücklauf 8 ihr Retentat bildet. Da die erste Membrantrenneinheit 6 das Katalysatorsystem deutlich langsamer passieren lässt als die übrigen Bestandteile des Hydroformylierungsaustrags 4, reichert sich im Reaktorrücklauf 8 das Katalysatorsystem an. Die Membrantrenneinheit 6 wird vorzugsweise so gefahren, dass sich etwa drei Viertel des aus dem Hydroformylierungsreaktors 1 ausgetragenen Katalysatorsystems im Reaktorrücklauf 8 findet. Der Rückhalt der ersten Membrantrenneinheit beträgt hinsichtlich des Katalysatorsystems somit 75%. Dafür sind folgende Betriebsparameter einzuhalten: Die Membrantemperatur liegt zwischen 20 und 160°C, bevorzugt zwischen 25 und 90°C und besonders bevorzugt zwischen 30 und 60°C. In den Konzentratloops kann eine um 10 bis 30 K höhere Temperatur vorteilhaft sein. Die transmembrane Druckdifferenz liegt zwischen 1 und 10 MPa, bevorzugt zwischen 1.5 und 5 MPa. Besonders bevorzugt wird die Membran bei etwa 2.5 bis 3.5 MPa Transmembrandruck betrieben. Die bevorzugt eingesetzte Membranmodulbauweise ist das Spiralwickelelement.

Vorzugsweise werden Membranen eingesetzt, die eine trennaktive Schicht aus einem Material, ausgewählt aus Celluloseacetat, Cellulosetriacetat, Cellulosenitrat, regenerierter Cellulose, Polyimiden, Polyamiden, Polyetheretherketonen, sulfonierten Polyetheretherketonen, aromatischen Polyamiden, Polyamidimiden, Polybenzimidazolen, Polybenzimidazolone, Polyacrylnitril, Polyarylethersulfone, Polyester, Polycarbonaten, Polytetrafluorethylen, Polyvinylidenfluorid, Polypropylen, endständig oder seitenständig organomodifiziertem Siloxan, Polydimethylsiloxan, Silicone, Polyphosphazene, Polyphenylsulfide, Polybenzimidazole, 6.6 Nylon®, Polysulfone, Polyaniline, Polypropylene, Polyurethane, Acrylonitril/Glycidylmethacrylat (PANGMA), Polytrimethylsilylpropyne, Polymethylpentyne, Polyvinyltrimethylsilan, Polyphenylenoxid, alpha-Aluminiumoxide, gamma-Aluminiumoxide, Titaniumoxide, Siliciumoxide, Zirconiumoxide, mit Silanen hydrophobisierte keramische Membranen, wie sie in EP 1 603 663 B1 beschrieben sind, Polymere mit intrinsischer Mikroporosität (PIM) wie PIM-1 und anderen, wie sie z. B. in EP 0 781 166 und in "Membranes" von I. Cabasso, Encyclopedia of Polymer Sience and Technlogy, John Wiley and Sons, New York, 1987, beschrieben werden, aufweisen. Die oben genannten Stoffe können insbesondere in der trennaktiven Schicht ggf. durch Zugabe von Hilfsstoffen vernetzt vorliegen oder als so genannte Mixed Matrix Membranen mit Füllstoffen wie z.B. Carbon Nano Tubes, Metal Organic Frameworks oder Hollow Spheres sowie Partikel von anorganischen Oxiden oder anorganische Fasern, wie z. B. Keramik- oder Glasfasern versehen sein.

Besonders bevorzugt werden Membranen eingesetzt, die als trennaktive Schicht eine Polymerschicht aus endständig oder seitenständig organomodifiziertem Siloxan, Polydimethylsiloxan oder Polyimid aufweisen, die aus Polymeren mit intrinsischer Mikroporosität (PIM) wie PIM-1 aufgebaut sind, oder wobei die trennaktive Schicht über eine hydrophobierte keramische Membran aufgebaut ist.

Ganz besonders bevorzugt werden Membranen aus endständig oder seitenständig organomodifiziertem Siloxanen oder Polydimethylsiloxanen eingesetzt. Solche Membranen sind kommerziell erhältlich.

Neben den oben genannten Materialien können die Membranen weitere Materialien aufweisen. Insbesondere können die Membranen Stütz- oder Trägermaterialien aufweisen, auf die die trennaktive Schicht aufgebracht ist. Bei solchen Verbundmembranen liegt neben der eigentlichen Membrane noch ein Stützmaterial vor. Eine Auswahl von Stützmaterialien beschreibt EP 0 781 166, auf welches explizit verwiesen wird.

Eine Auswahl von kommerziell erhältlicher Lösungsmittel stabiler Membranen sind die MPF und Selro Serien von Koch Membrane Systems, Inc., unterschiedliche Typen von Solsep BV, die Starmem™ Serie von Grace/UOP, die DuraMem™ und PuraMem™ Serie von Evonik Industries AG, die Nano-Pro Serie von AMS Technologies, die HITK-T1 von IKTS, sowie oNF-1, oNF-2 und NC-1 von der GMT Membrantechnik GmbH und die inopor®nano Typen der Inopor GmbH.

Das Retentat der ersten Membrantrenneinheit 6 - hier als Reaktorrücklauf 8 oder auch als primäres Rezyklat bezeichnet - enthält neben der hohen Rhodiumkonzentration die übrigen Stoffe des Hydroformylierungsaustrags, nämlich Aldehyd, Olefin, gelöstes Synthesegas, weitere Leichtsieder und Hochsieder. Der Reaktorrücklauf 8 wird in den Hydroformylierungsreaktor 1 zurückgeführt. Der Reaktorrücklauf braucht dabei nicht - wie in der Zeichnung vereinfacht dargestellt - zusammen mit dem Frisch-Olefin 2 und dem frischen Synthesegas 3 in den Reaktor 1 eingespeist werden. Es ist durchaus denkbar, diese 3 Ströme an unterschiedlichen Stellen getrennt in den Hydroformylierungsreaktor 1 einzuspeisen.

Um zu verhindern, dass das Katalysatorsystem in der ersten Membrantrenneinheit 6 seine Aktivität verliert, wird dieser Membrantrennschritt unter Einhaltung eines Mindest-CO-Partialdampfdruckes durchgeführt. Dieser sollte, wie in EP1931472B1 geschildert, mindestens 100 kPa betragen. Aus diesem Grund erfolgt die Entspannung in Entgaser 9a nicht vollständig sondern nur bis auf 0.5 MPa. Das gelöste Synthesegas soll nämlich erst hinter der Membran entfernt werden. Hierfür wird der Produktstrom 7 in einem zweiten Entgaser 9b auf Atmosphärendruck entspannt. Das im Permeat der ersten Membrantrenneinheit verbliebene Synthesegas entweicht dabei vollständig und wird aus der Anlage abgeführt.

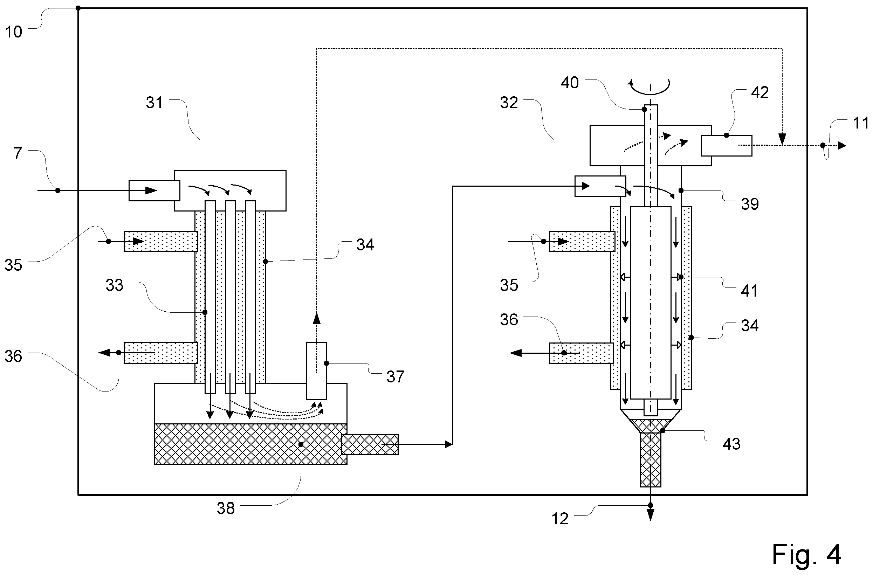

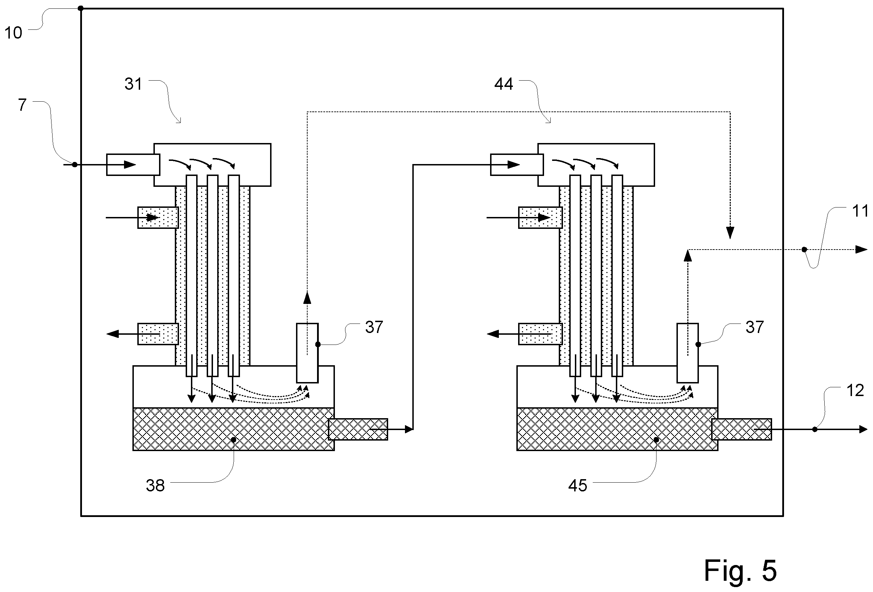

Sodann wird der entgaste Produktstrom 7 in eine thermische Trenneinheit 10 überführt. Dabei handelt es sich einfachsten falls um eine Destillationskolonne, bevorzugt jedoch um eine Kombination aus einem Dünnschichtverdampfer und einem Fallfilmverdampfer (vgl. Figur 4) oder einer Kombination von zwei oder drei Fallfilmverdampfern (vgl. Figur 5).

In der thermischen Trenneinheit 10 wird der Produktstrom 7 unter Wärmeeinwirkung verdampft. Hierfür wird am Fallfilmverdampfer eine Sumpftemperatur von 90°C eingestellt; die Sumpftemperatur am Dünnschichtverdampfer beträgt 100°C. Unterstützt wird die Verdampfung durch ein angelegtes Vakuum von jeweils etwa 30 hPa. Auf diese Weise verdampft mehr als 90 % der mit dem Produktstrom 7 in die thermische Trenneinheit 10 eingebrachten Masse. Diese dampfförmige Masse bildet das Kopfprodukt 11 der thermischen Trenneinheit. Da die eingebrachten Komponenten unterschiedliche Siedepunkte aufweisen, findet durch die Verdampfung nicht nur eine rein quantitative Trennung des Produktstroms 7 statt sondern auch eine qualitative: Aufgrund des niedrigeren Siedepunkts werden Aldehyd, Alkohol und die übrigen Leichtsieder bevorzugt im Kopfprodukt 11 angereichert. Die nicht verdampften Komponenten bilden ein flüssiges Sumpfprodukt 12, welches im Wesentlichen aus Hochsiedern, Aldehyd und Katalysatorsystem besteht, wobei Aldehyd und Hochsieder etwa denselben Gewichtsanteil ausmachen. Die Betriebsbedingungen der thermischen Trenneinheit sind so gewählt, dass sich vorzugsweise 95 % der mit dem Produktstrom 7 eingebrachten Aldehyde im Kopfprodukt 11 finden. Maximal 5 % der mit dem Produktstrom 7 eingebrachten Aldehyde verbleiben im Sumpf 12.

Der als Kopfprodukt 11 abgezogene, die Wertprodukte enthaltende, große Massenstrom wird sodann durch einen ersten Adsorber 13 gefahren. Die Funktion des Adsorbers 13 besteht darin, Restmengen von Katalysator, insbesondere Edelmetall, welches in Tröpfchen mit dem Dampf mitgerissen wurde, aufzufangen. Dies gelingt durch den Einsatz eines herkömmlichen Adsorptionsmittels wie Aktivkohle, Silicate oder Aluminiumoxide, welche als Festbett eingesetzt werden. Die Adsorption wird bei einer Temperatur zwischen 30 und 140 °C und Raumbelastungen von 0,01 bis 5 1/h durchgeführt.

Das nun vollständig von Katalysatorfracht gereinigte Kopfprodukt 11 wird nun einer Hydrierung 14 zugeführt. Die Hydrierung erfolgt in zwei hintereinander geschalteten Hydrierreaktoren. Der erste Reaktor wird in Schlaufenfahrweise betrieben, der zweite im geraden Durchlauf. Die Hydrierung erfolgt in der Flüssigphase in einem Temperaturbereich von 120 bis 220°C, bevorzugt bei einer Temperatur von 160°C und zwar adiabatisch. Der Druck beträgt 1.5 bis 30 MPa. Die Hydrierung erfolgt in einem heterogenen Festbettkatalysator wie zum Beispiel Kupfer-, Kobalt-, Kupfer/Nickel-, Kupfer/Chrom-, Kupfer/Chrom/Nickel-, Zink/Chrom-, Nickel/Molybdän-Katalysatoren, die gegebenenfalls noch weitere Elemente aufweisen können. Details zur Ausgestaltung einer geeigneten Hydrierung sind in EP0987240B1 und EP0987241B1, sowie in DE102008007080A1 beschrieben.

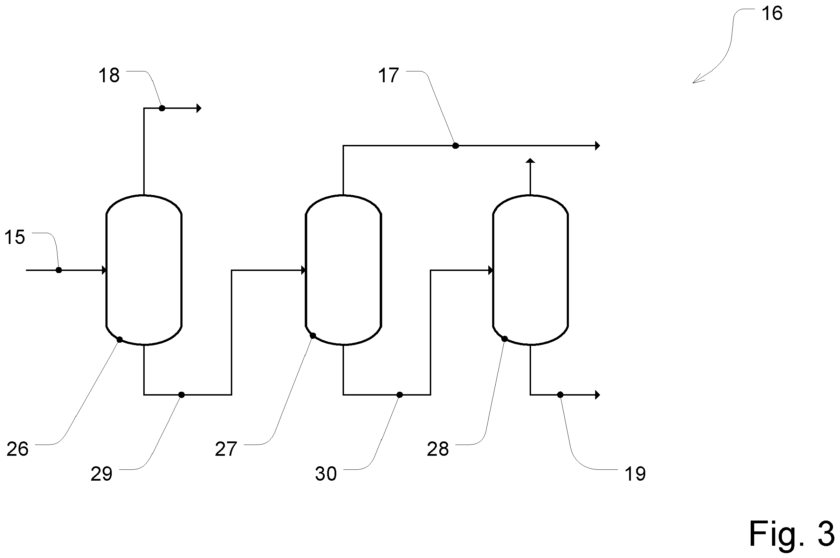

Das aus der Hydrierung 14 abgezogene Hydrierungsgemisch 15 umfasst nun im Wesentlichen Alkohol, Alkane (aus nicht umgesetzten Olefinen) sowie hydrierte Leichtsieder und Hochsieder. Das Hydrierungsgemisch 15 wird sodann einer thermischen Aufarbeitung 16 zugeführt und dort in eine alkoholreiche Fraktion 17, eine Leichtsiederfraktion 18 und einer Hochsiederfraktion 19 aufgeteilt. Die alkoholreiche Fraktion 17 stellt dabei das eigentliche Wertprodukt des erfindungsgemäßen Verfahrens dar. Die Leichtsieder 18 und Hochsieder 19 stellen Nebenprodukte dar, die sich für untergeordnete Zwecke vermarkten lassen. Die thermische Aufarbeitung 16 der drei Fraktionen 17, 18 und 19 aus dem Hydrierungsgemisch 15 wird anhand der Figur 3 noch näher erläutert werden.

Falls der Umsatz der Alkene in der Hydroformylierung nicht vollständig ist, werden sich die im Kopfprodukt 11 der thermischen Trenneinheit 10 eine größere Menge nicht umgesetzte Alkene finden. Damit diese nicht in der Hydrierung 14 verloren gehen, kann die Hydrierung auch hinter der thermischen Aufarbeitung angeordnet werden, und zwar einmal für die alkoholreiche Fraktion 17 (die in diesem Fall eher aldehydreich ist) und einmal für die Hochsiederfraktion 19. Die Leichtsiederfraktion 18 enthält dann die nicht umgesetzten Alkene und kann in den Hydroformylierungsreaktor 1 zurück geführt werden (nicht in dieser Figur dargestellt). Das Kopfprodukt 11 wird bei einer Alkenrückführung somit nicht vollständig, sondern nur teilweise der Hydrierung zugeführt, die Alkene werden zuvor abgetrennt und zurückgeführt.

Wie bereits erwähnt, finden sich im Sumpfprodukt 12 der thermischen Trenneinheit 10 im Wesentlichen die Hochsieder, geringere Mengen Aldehyd und Katalysator. Der Massenstrom des Sumpfproduktes 12 ist deutlich kleiner als der des Kopfproduktes. Beträgt der Produktstrom 30 Tonnen pro Stunde und wird die Maßgabe eingehalten, dass 90 % der eingetragenen Masse die thermische Trenneinheit 10 über Kopf verlässt, beträgt der Massenstrom des Sumpfproduktes 12 lediglich 3 t pro Stunde, also 1/9 des Kopfproduktes.

Das Sumpfprodukt 12 wird nun einer zweiten Membrantrenneinheit 20 aufgegeben. Dort wird das Sumpfprodukt 12 aufgetrennt in ein Permeat 21 und ein Retentat 22, wobei das Katalysatorsystem im Retentat 22 angereichert wird, da die zweite Membrantrenneinheit 20 das Katalysatorsystem bevorzugt zurückhält. Dank des geringen Massenstroms, den die zweite Membrantrenneinheit 20 im Vergleich zur ersten Membrantrenneinheit 6 aufzutrennen hat, ist es möglich, den im Sumpfprodukt 12 enthaltenen Katalysator nahezu vollständig zurückzuhalten und im Retentat 22 anzureichern. Dies gelingt insbesondere dann, wenn ein Membranmaterial gewählt wird, welches im besonderen Maße für Hochsieder durchlässig ist und folglich die Hochsieder im Permeat 21 anreichert. Retentat 22 besteht dann im Wesentlichen aus Aldehyd und Katalysator.

Die Trennung in der zweiten Membrantrenneinheit 20 erfolgt bei einer Temperatur zwischen 20 und 160°C, bevorzugt zwischen 25 und 90°C und besonders bevorzugt zwischen 30 und 60°C. Die transmembrane Druckdifferenz liegt zwischen 1 bis 10 MPa, bevorzugt zwischen 1.5 und 5 MPa. Besonders bevorzugt wird die Membran bei etwa 2.5 bis 3.5 MPa Transmembrandruck betrieben. Die bevorzugt eingesetzte Membranmodulbauweise ist das Spiralwickelelement.

Die erste und die zweite Membraneinheit können mit denselben oder unterschiedlichen Membranmaterialien arbeiten.

Als Membranmaterial kommen für die zweite Membrantrenneinheit 20 die im Folgenden beschriebenen Materialklassen in Betracht:

Vorzugsweise werden innerhalb der zweiten Membrantrenneinheit Membranen eingesetzt, die eine trennaktive Schicht aus einem Material, ausgewählt aus Celluloseacetat, Cellulosetriacetat, Cellulosenitrat, regenerierter Cellulose, Polyimiden, Polyamiden, Polyetheretherketonen, sulfonierten Polyetheretherketonen, aromatischen Polyamiden, Polyamidimiden, Polybenzimidazolen, Polybenzimidazolone, Polyacrylnitril, Polyarylethersulfone, Polyester, Polycarbonaten, Polytetrafluorethylen, Polyvinylidenfluorid, Polypropylen, endständig oder seitenständig organomodifiziertem Siloxan, Polydimethylsiloxan, Silicone, Polyphosphazene, Polyphenylsulfide, Polybenzimidazole, 6.6 Nylon, Polysulfone, Polyaniline, Polypropylene, Polyurethane, Acrylonitril/Glycidylmethacrylat (PANGMA), Polytrimethylsilylpropyne, Polymethylpentyne, Polyvinyltrimethylsilan, Polyphenylenoxid, $\alpha$-Aluminiumoxide, $\gamma$-Aluminiumoxide, Titaniumoxide, Siliciumoxide, Zirconiumoxide, mit Silanen hydrophobisierte keramische Membranen, wie sie in EP 1 603 663 B1 beschrieben sind, Polymere mit intrinsischer Mikroporosität (PIM) wie PIM-1 und anderen, wie sie z. B. in EP 0 781 166 und in "Membranes" von I. Cabasso, Encyclopedia of Polymer Sience and Technlogy, John Wiley and Sons, New York, 1987, beschrieben werden, aufweisen. Die oben genannten Stoffe können insbesondere in der trennaktiven Schicht ggf. durch Zugabe von Hilfsstoffen vernetzt vorliegen oder als so genannte Mixed Matrix Membranen mit Füllstoffen wie z.B. Carbon Nano Tubes, Metal Organic Frameworks oder Hollow Spheres sowie Partikel von anorganischen Oxiden oder anorganische Fasern, wie z. B. Keramik- oder Glasfasern versehen sein.

Besonders bevorzugt werden Membranen eingesetzt, die als trennaktive Schicht eine Polymerschicht aus endständig oder seitenständig organomodifiziertem Siloxan, Polydimethylsiloxan oder Polyimid aufweisen, die aus Polymeren mit intrinsischer Mikroporosität (PIM) wie PIM-1 aufgebaut sind, oder wobei die trennaktive Schicht über eine hydrophobierte keramische Membran aufgebaut ist.

Eine detaillierte Beschreibung solcher Membranen zum Einsatz in der Hochsiederausschleusung findet sich in EP2401078A1.

Ganz besonders bevorzugt werden Membranen aus endständig oder seitenständig organomodifiziertem Siloxanen oder Polydimethylsiloxanen eingesetzt. Solche Membranen sind kommerziell erhältlich.

Neben den oben genannten Materialien können die Membranen weitere Materialien aufweisen. Insbesondere können die Membranen Stütz- oder Trägermaterialien aufweisen, auf die die trennaktive Schicht aufgebracht ist. Bei solchen Verbundmembranen liegt neben der eigentlichen Membrane noch ein Stützmaterial vor. Eine Auswahl von Stützmaterialien beschreibt EP0781166, auf welches explizit verwiesen wird.

Eine Auswahl von kommerziell erhältlicher Lösungsmittel stabiler Membranen sind die MPF und Selro Serien von Koch Membrane Systems, Inc., unterschiedliche Typen von Solsep BV, die Starmem™ Serie von Grace/UOP, die DuraMem™ und PuraMem™ Serie von Evonik Industries AG, die Nano-Pro Serie von AMS Technologies, die HITK-T1 von IKTS, sowie oNF-1, oNF-2 und NC-1 von der GMT Membrantechnik GmbH und die inopor®nano Typen der Inopor GmbH.

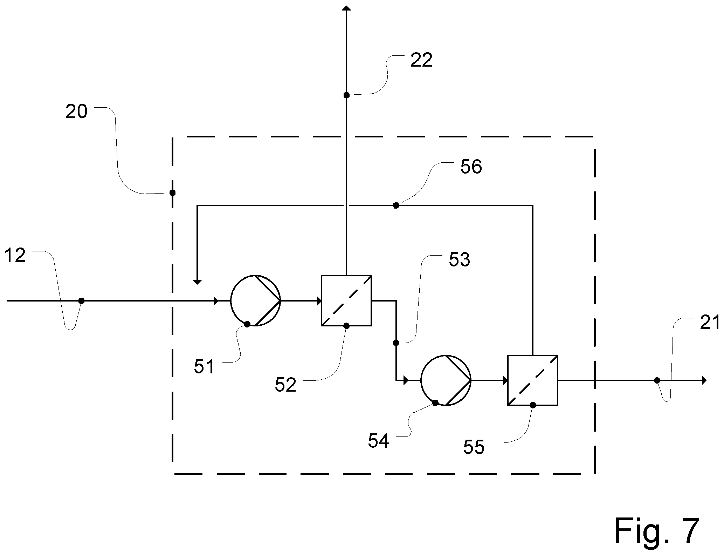

Die zweite Membrantrenneinheit 20 wird als mehrstufige Verstärkungskaskade ausgeführt. Diese Membranverschaltung wird anhand der Figur 7 näher erläutert werden. Für das Verständnis der Funktion der zweiten Membrantrenneinheit 20 genügt die Annahme, dass es sich um eine einfache Membran handelt.

Das von der zweiten Membrantrenneinheit 20 abgezogene Retentat 22 wird in einem Wärmetauscher 23 auf etwa 40 bis 50 °C abgekühlt und sodann mit dem ebenfalls abgekühlten Hydroformylierungsaustrag 4 vermischt und in die erste Membrantrenneinheit 6 zurückgeführt. Das Zurückführen des sekundären Rezyklats (Retentat 22) in die erste Membrantrenneinheit 6 hinter dem Hydroformylierungsreaktor 1 eröffnet den entscheidenden Vorteil, dass regelungstechnische Interferenzen zwischen der Regelung der zweiten Membrantrenneinheit 20 und dem Hydroformylierungsreaktor 1 reduziert werden. Auch wird verhindert, dass unnötigerweise Aldehyd mit dem Retentat 22 in die Reaktion zurückgefahren wird und deren Ausbeute schmälert. Die über den sekundären Rücklauf 22 zurückgeführten Katalysatorbestandteile werden größtenteils von der ersten Membrantrenneinheit 6 wieder abgeschöpft und über den primären Reaktorrücklauf 8 in den Reaktor 1 zurückgeführt.

Das weitestgehend aus Hochsiedern und Rest-Aldehyd bestehende Permeat 21 der zweiten Membrantrenneinheit 20 wird durch einen zweiten Adsorber 24 geleitet, um Restmengen von Katalysator aufzunehmen und zu sichern. Zur Abscheidung von edlen Katalysatormetallen aus dem flüssigen Permeat 21 wird die Adsorption bei einer Temperatur von 30 bis 140 °C und Raumbelastungen von 0,01 bis 5 1/h durchgeführt. Bevorzugt wird das Adsorptionsmittel als Festbett eingesetzt.

Als Adsorptionsmittel können insbesondere Aktivkohleoberflächenreiche Polykieselsäuren wie Silicagele (Kiesel-Xerogele), hochdisperse Kieselsäure, oberflächenreiche Aluminiumoxide und Aluminiumoxidhydrate als auch gebrauchte oder neue (Hydrier-)Katalysatoren eingesetzt werden.

Als besonders vorteilhafte Adsorbentien haben sich chemisch modifizierte Silica-Materialien erwiesen, wie sie in WO 2006013060 A1 offenbart sind. Derartige Adsorptionsmittel sind unter der Artikelbezeichnung Mercaptoalkylmodified Silica, Type Rh H3, Batch No. 09-S26-001 der Firma PhosphonicS Ltd, 114 Milton Park, Abingdon, OXON, OX14 4SA, United Kingdom erhältlich.

Das nunmehr vollständig von Katalysatorrückständen adsorptiv gereinigte Permeat 21 wird nun gemeinsam mit dem ebenfalls adsorptiv gereinigten Kopfprodukt 11 der Hydrierung 14 zugeführt. Alternativ wäre es denkbar, Kopfprodukt 11 und Permeat 21 anstelle einer gemeinsamen Hydrierung 14 getrennten Hydrierungsreaktionen zuzuführen (nicht dargestellt).

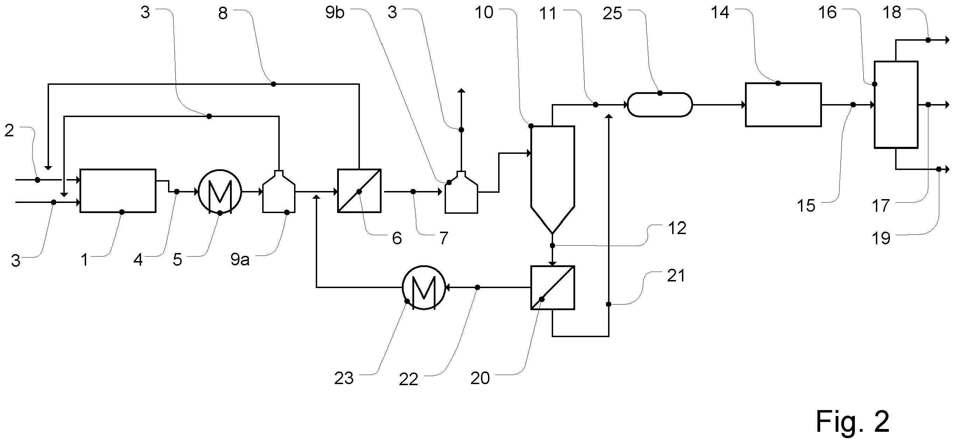

Figur 2 zeigt eine Variante der Anlage aus Figur 1, bei der das Permeat 21 der zweiten Membrantrenneinheit 20 und das Kopfprodukt 11 der thermischen Trenneinheit 10 durch einen gemeinsamen Adsorber 25 gefahren werden und sodann der Hydrierung 14 unterworfen werden. Durch Verwendung eines gemeinsamen Adsorbers 25 kann Adsorbens eingespart werden, was die Betriebskosten der Anlage senkt. Die Betriebsbedingungen und das Adsorbens werden bei dieser Variante so gewählt wie eben zu dem zweiten Adsorber 24 beschrieben.

Figur 3 zeigt die thermische Aufarbeitung 16 im Detail. Diese besteht aus einer seriellen Anordnung dreier Destillationskolonnen 26, 27 und 28, welche bei Normaldruck oder bei vermindertem Druck betrieben werden. Das Hydrierungsgemisch 15 wird in die erste Kolonne 26 eingespeist. Das Hydrierungsgemisch wird darin aufgetrennt in eine über Kopf abgezogene Leichtsiederfraktion 18 und einer Sumpffraktion 29, die im Wesentlichen aus Hochsiedern und Alkohol besteht. Die erste Destillationskolonne 26 weist von 20 bis 70, bevorzugt von 28 bis 65 theoretische Trennstufen auf. Die Temperatur in der ersten Destillationskolonne 26 wird vorzugsweise so eingestellt, dass die Kopftemperatur 85 bis 110 °C, bevorzugt 95 bis 100 °C und die Sumpftemperatur 175 bis 200 °C, bevorzugt 185 bis 193 °C beträgt.

Die Sumpffraktion 29 der ersten Kolonne 26 wird in die zweite Destillationskolonne 27 eingespeist. In dieser wird über Kopf die alkoholreiche Fraktion 17 abgenommen. Diese besteht bevorzugt mehr als 98 % aus dem Zielalkohol. Im Sumpf 30 der zweiten Destillationskolonne 27 fällt ein Gemisch aus Hochsiedern und Restalkohol an. Zur Erfüllung dieser Trennaufgabe weist die zweite Destillationskolonne 27 von 8 bis 35, bevorzugt von 10 bis 30 theoretische Stufen auf. Die Temperatur in der zweiten Destillationskolonne 27 wird vorzugsweise so eingestellt, dass die Kopftemperatur 150 bis 180 °C, bevorzugt 160 bis 170 °C und die Sumpftemperatur 180 bis 205 °C, bevorzugt 185 bis 195 °C beträgt.

Der Sumpf 30 der zweiten Destillationskolonne 27 wird abschließend in eine dritte Kolonne 28 gefahren, an deren Sumpf die Hochsiederfraktion 19 anfällt. An ihrem Kopf gehen Restmengen an Alkohol ab, die mit der alkoholreichen Fraktion 17 vermischt werden. Die dritte Destillationskolonne 28 weist 15 bis 35, bevorzugt von 20 bis 30 theoretische Trennstufen auf. Die Temperatur in der dritten Destillationskolonne wird vorzugsweise so eingestellt, dass die Kopftemperatur von 95 bis 120 °C, bevorzugt 100 bis 110 °C und die Sumpftemperatur von 160 bis 190 °C, bevorzugt 165 bis 175 °C beträgt.

Die drei Fraktionen 17, 18 und 19 werden aus dem Prozess ausgeschleust und vermarktet.

Figur 4 zeigt den internen Aufbau der thermischen Trenneinheit 10. Diese wird gebildet von zwei hintereinander geschalteten thermischen Trennapparaten, nämlich einem Fallfilmverdampfer 31 und einem Dünnschichtverdampfer 32. Der Fallfilmverdampfer 31 ist von herkömmlicher technischer Bauart. Der flüssige Produktstrom 7 strömt am Kopf des Fallfilmverdampfers 31 ein und wird dort auf eine Vielzahl vertikal verlaufende Fallrohre 33 verteilt. Die Fallrohre 33 sind von einem mit Mitteldruckdampf beheizten Heizmantel 34 umgeben. Bei dem Mitteldruckdampf handelt es sich um als Wärmemedium eingesetzten Wasserdampf, der mit den Prozesschemikalien nicht reagiert. Sein Druck liegt zwischen 1.2 und 2.4 MPa, je nach Standortbedingungen. Der Mitteldruckdampf tritt durch einen Dampfeinlass 35 in den Heizmantel 34 ein, gibt seine Wärme über die Wandung der Fallrohre 33 auf den Produktstrom 7 ab und tritt abgekühlt über einen Dampfauslass 36 wieder aus. Durch die Fallrohre 33 läuft der flüssige Produktstrom 7 der Schwerkraft folgend hinab und wird dabei von dem heißen Dampf (etwa 120 °C) aufgeheizt. Beim Austritt am Fuß der Fallrohre 33 sind die bei 120 °C siedenden Komponenten des Produktstroms 7 weitestgehend verdampft. Hierbei ist zu berücksichtigen, dass in dem Fallfilmverdampfer 31 ein Unterdruck von 3 und 500 hPa herrscht. Die verdampften Anteile des Produktstroms 7 verlassen den Fallfilmverdampfer 31 über einen Gasaustritt 37. Die nicht verdampften Komponenten sammeln sich im Sumpf 38 und werden von dort in den Dünnschichtverdampfer 32 geleitet.

Der Dünnschichtverdampfer 32 verfügt in ähnlicher Weise wie der Fallfilmverdampfer 31 über einen Mitteldruckdampf beheizten Heizmantel 34, durch den der Prozessdampf durch einen Dampfeinlass 35 einströmt und durch abgekühlt durch einen Dampfauslass 36 wieder verlässt. Mit dem Dampf wird ein Tubus 39 von außen beheizt, an dessen Innenseite die bisher nicht verdampften Anteile des Produktstroms 7 aus dem Sumpf 38 des Fallfilmverdampfers 31 entlang läuft. Koaxial innerhalb des Tubus 39 angeordnet ist ein Rotor 40, der sich um die Längsachse des Dünnschichtverdampfers 32 dreht. Er ist mit einer Vielzahl von Wischorganen 41 versehen, die den flüssigen Feed in eine dünne Schicht auf der Innenseite des Tubus 39 verteilen. Die dabei verdampfenden Anteile verlassen den Dünnschichtverdampfer 32 über einen Gasaustritt 42 und werden sodann mit den verdampften Komponenten aus dem Fallfilmverdampfer 31 (ex Gasaustritt 37) zu dem Kopfprodukt 11 der thermischen Trenneinheit 10 vereinigt. Auf diese Weise wird etwa 90 % der mit dem Produktstrom 7 in die thermische Trenneinheit 10 eingebrachten Masse verdampft und als Kopfprodukt 11 abgezogen.

Die verbleibenden 10 % des eingebrachten Produktstroms 7 verlässt die thermische Trenneinheit 10 flüssig, nämlich vom Sumpf 43 des Dünnschichtverdampfers, wo sich die innerhalb des Tubus 39 nicht verdampften Anteile des Zulaufs des Dünnschichtverdampfers 32 ansammeln. Der Sumpf 43 entspricht mithin dem Sumpfprodukt 12 der thermischen Trenneinheit 10.

In Figur 5 ist eine alternative Ausführungsform der thermischen Trenneinheit 10 dargestellt. Diese besteht aus zwei hintereinander geschalteten Fallfilmverdampfer 31, 44. Beide Fallfilmverdampfer 31, 44 entsprechen dem in Figur 4 gezeigten Fallfilmverdampfer 31 und brauchen deswegen nicht näher erläutert zu werden. Ihre jeweiligen Gasaustritte 37 sind zum Kopfprodukt 11 der thermischen Trenneinheit 10 vereinigt. Das Sumpfprodukt 12 der thermischen Trenneinheit 10 wird vom Sumpf 45 des zweiten Fallfilmverdampfers 44 abgezogen. Der Sumpf 38 des ersten Fallfilmverdampfers 31 dient als Zulauf für den zweiten Fallfilmverdampfer 44. In derselben Weise ist es möglich, drei Fallfilmverdampfer hintereinander zu schalten (nicht dargestellt).

Figur 6 zeigt den prinzipiellen Aufbau der ersten Membrantrenneinheit 6. Bei der ersten Membrantrenneinheit 6 handelt es sich um eine zweistufige Abtriebskaskade. Dieser wird ein Gemisch aus dem entgasten Hydroformulierungsaustrag 4 des Reaktors und dem Retentat 22 der zweiten Membrantrennstufe mittels einer Pumpe 46 einer ersten Stufe 47 aufgegeben. Das Permeat der ersten Stufe 47 entspricht dem resultierenden Permeat der ersten Membrantrenneinheit 6 und damit dem Produktstrom 7 der Anlage.

Das Retentat 48 der ersten Stufe 47 wird ohne eine weitere Druckerhöhung einer zweiten Stufe 49 aufgegeben. Das Retentat 8 der zweiten Stufe 49 entspricht dem resultierenden Retentat der ersten Membrantrenneinheit 6 und wird als Reaktorrücklauf 8 vor den Hydroformulierungsreaktor 1 zurückgeführt. Das Permeat 50 der zweiten Stufe 49 wird mit dem Feed der ersten Membrantrenneinheit vermischt und über die Pumpe 46 wieder der ersten Stufe 47 zugeführt. Das Permeat 50 der zweiten Stufe 49 entspricht somit der internen Permeatrückführung der als Abtriebskaskade ausgeführten Membrantrenneinheit.

Figur 7 zeigt den internen Aufbau der zweiten Membrantrenneinheit 20. Diese ist als zweistufige Verstärkungskaskade ausgeführt. Als Feed für die zweite Membrantrenneinheit 20 dient das Sumpfprodukt 12 der thermischen Trenneinheit 10. Es wird mittels einer ersten Druckerhöhungspumpe 51 auf einen Druck von etwa 3 MPa vorgespannt und einer ersten Stufe 52 aufgegeben. Das Retentat der ersten Stufe 52 entspricht dem resultierenden Retentat der zweiten Membrantrenneinheit 20 und verlässt als Retentat 22 / sekundäres Rezyklat die zweite Membrantrennstufe 20 und wird mit dem entgasten Hydroformulierungsaustrag 4 vermischt in die erste Membrantrenneinheit 6 zurückgeführt.

Das Permeat 53 der ersten Stufe 52 wird mittels einer zweiten Druckerhöhungspumpe 54 wieder auf einen Druck von etwa 3 MPa gebracht, um den Transmembrandruck der ersten Stufe 52 wieder auszugleichen. Sodann erfolgt die zweiten Stufe 55 der Membrantrennung. Das dabei anfallende Permeat 21 entspricht dem resultierenden Permeat 21 der zweiten Membrantrenneinheit 20. Es wird nach adsorptiver Reinigung der Hydrierung zugeführt. Das Retentat 56 der zweiten Stufe 55 wird mit dem Feed der zweiten Membrantrennstufe 20 (= Sumpfprodukt 12) vermischt und über die erste Druckerhöhungspumpe 51 in die erste Stufe 52 zurückgeführt. Das Retentat 56 der zweiten Stufe stellt somit die interne Retentatrückführung der Verstärkungskaskade dar.

**Beispiele**

Varianten der erfindungsgemäßen Aufarbeitung des Hydroformylierungsaustrags sollen nun anhand von Simulationen verglichen werden. Aufgrund der komplexen Anlagenstruktur ist die Simulation das Mittel der Wahl.

Betrachtet wird die Herstellung von $C_9$-Alkoholen aus $C_8$-Olefinen.

Modell der Hydroformylierung

In der Simulation wurde die Hydroformylierung des $C_8$-Olefingemisches Dibuten vereinfacht über einen formalkinetischen Ansatz beschrieben. Dabei wurden die folgenden Reaktionen berücksichtigt. Hauptreaktion ist die Hydroformylierung von Dibuten mit Synthesegas (CO + H2) zu dem $C_9$-Aldehyd Nonanal (INAL) gemäß Reaktion 1:

Reaktion 1: Dibuten + CO + H2 → INAL

Weiterhin wird als Folgereaktion 2 die Hydrierung vom Aldehyd INAL zum Alkohol Isononanol (INA) berücksichtigt:

Reaktion 2: INAL + H2 → INA

Bei der Simulation des Hydroformylierungsprozesses, bei dem Nanofiltration eingesetzt wird, ist insbesondere von Interesse, wie sich mögliche Hochsieder im Katalysatorkreislauf anreichern. Die Hochsiederbildung setzt sich aber aus einer Vielzahl an unbekannten Reaktionen zusammen. Um das Reaktionsystem so einfach wie möglich zu halten, wird in dem Kinetik-Modell deshalb nur eine weitere Reaktion berücksichtigt, um die Hochsiederbildung zu modellieren. Entsprechend gibt es in der Simulation auch nur eine schwersiedende Komponente, die stellvertretend das eigentliche Hochsiedergemisch darstellt, das bei der Hydroformylierung entsteht. Die Hochsieder werden in der Simulation durch Dinonylether (DiEther) abgebildet. Der Dinonylether entsteht gemäß Reaktion 3 von Nonanal (INAL) und Nonanol (INA):

Reaktion 3: INA +INAL + H2 → H2O + DiEther

Im Prinzip ist die Auswahl der Hochsiederreaktion willkürlich. So kann die Etherbildung in Reaktion 3 auch durch eine andere Reaktion ersetzt werden, bei der kein Wasser (H2O) gebildet wird, z.B. eine Acetalbildung.

Um die Abhängigkeiten der Reaktionen von den verschiedenen Größen abzubilden wurden folgende Gleichungen für die Modellierung der Reaktionsraten (in kmol m$^{-3}$ min$^{-1}$)$r_i$, i=1,...,3 eingesetzt:

$$r_1 = c_{total} k_1 \left( x_{Dibuten}^{n_1} - \left( \frac{x_{Nonanal}}{k_{ggw} p^2} \right)^{n_1} \right) c_{Rh}^{n_{Rh}} k_{Li} k_p \tag{1}$$

$$r_2 = c_{total} k_2 x_{Nonanal} c_{Rh}^{n_{Rh}} \tag{2}$$

$$r_3 = c_{total} k_3 x_{Nonanal} x_{Nonanaol} \tag{3}$$

Hierbei ist $c_{total}$ die gesamte Stoffmengenkonzentration [kmol / m³], $x_j$ ist der molare Anteil der Komponente i, p ist der Druck in bar, und $c_{Rh}$ die Rh-Konzentration in ppm. Die Abhängigkeit von der Rh-Konzentration wird über den Exponenten nRh abgebildet; $n_1$ ist die Ordnung der ersten Reaktion. Die Reaktionsgeschwindigkeit $k_i$ wird mit dem Arrhenius-Ansatz modelliert:

$$k_i = k_{0,i} \exp\left( -\frac{E_{Ai}}{RT} \right) \tag{4}$$

Der Term $k_{Li}$ gibt die Abhängigkeit der Hydroformylierungsreaktion R1 von dem Verhältnis zwischen Ligand zu Rhodium wieder.

$$k_{Li} = 1 + \frac{k_{Li,1} X_{Li}}{1 + k_{Li,2} X_{Li}^2} \qquad (5)$$

$X_{Li}$ ist das molare Verhältnis zwischen Ligand und Rhodium. Die Druckabhängigkeit $k_p$ wird wiedergegeben durch:

$$k_p = \tanh\left(k_{p,0} \, p\right) \qquad (6)$$

Schließlich wird mit der Konstanten $k_{ggw}$ das (Pseudo-) Gleichgewicht zwischen Dibuten und Nonanal beschrieben. Die Werte aller Konstanten sind in Tabelle 1 zusammengefasst.

Tabelle 1: Werte der konstanten Reaktionsparameter

| Parameter | Wert | Parameter | Wert |
|---|---|---|---|
| $k_{0,1}$ | $13537 \text{ min}^{-1}$ | $E_{A,1}$ | 56037 kJ/kmol K |
| $k_{0,2}$ | $26810 \cdot 10^3 \text{ min}^{-1}$ | $E_{A,2}$ | 92703 kJ/kmol K |
| $k_{0,3}$ | $1840 \text{ min}^{-1}$ | $E_{A,3}$ | 50858 kJ/kmol K |
| $k_{Li,1}$ | 2,453 | $k_{Li,2}$ | 0,01342 |
| $k_{p,0}$ | $0,004975 \text{ bar}^{-1}$ | $k_{GGW}$ | $0,0601 \text{ bar}^{-2}$ |
| $n_1$ | 1,452 | $n_{Rh}$ | 0,6 |

Die Hydroformylierungsreaktion (R1) ist eigentlich keine Gleichgewichtsreaktion. Ergebnisse von Autoklavenversuche zeigen jedoch, dass bis zum Versuchende nach 6 h kein vollständiger Dibutenumsatz erreicht wird. Eine mögliche Erklärung hiefür ist, dass die langsamer reagierenden Di-Methylhexen-Isomere am Ende der Versuche noch nicht vollständig umgesetzt sind. Die hier modellierte einfache Formalkinetik unterscheidet jedoch nicht zwischen den verschiedenen Dibutenen. Durch die Einführung eines Pseudo-Gleichgewichts zwischen Dibuten und Nonanal kann der unvollständige Umsatz beschrieben werden. Die quadratische Druckabhängigkeit in Gleichung (1) ergibt sich aus der Pseudo-Gleichgewichtsbedingung:

$$k_{ggw}^* = \frac{x_{Nonanal,ggw}}{x_{Dibuten,ggw} x_{CO,ggw} x_{H_2,ggw}} \approx a \frac{x_{Nonanal,ggw}}{x_{Dibuten,ggw} p_{CO} p_{H_2}} \approx a^* \frac{x_{Nonanal,ggw}}{x_{Dibuten,ggw} p^2}, \quad k_{ggw} = \frac{k_{ggw}^*}{a^*} \quad (7)$$

Erst mit der quadratischen Druckabhängigkeit des Gleichgewichtsterms können die Versuchsergebnisse bei variierendem Druck zufriedenstellend durch die Kinetik nachgebildet werden.

Modell der organophilen Nanofiltration (Membrantrennung)

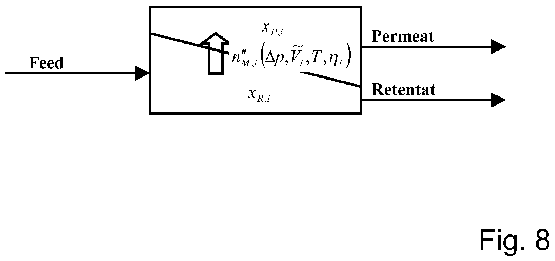

Die organophile Nanofiltration durch die Membran wird für die Simulation durch ein einfaches Modell abgebildet. In dem Modell wird der transmembrane Fluss als Funktion der Temperatur, des Transmembrandruckes und der Zusammensetzung auf Retentat- und Permeatseite berechnet. In dem vereinfachten Ansatz, das dem Modell zugrunde liegt, wird kein örtlich verteiltes Konzentrationsprofil berechnet und der Druckverlust beim Überströmen der Membran vernachlässigt. Durch die Annahme, dass auf der gesamten Membranfläche die Zusammensetzung am Ausgang der Membran vorliegt - und damit auch die treibende Konzentrationsdifferenz am Ausgang, wird die Trennwirkung der Membran in dem Modell unter- und die Fläche überschätzt. Wegen der Einfachheit der Gleichungen eignet sich das Membranmodul jedoch für ein erstes Screening der verschiedenen Verfahrensvarianten durch Simulation. Ein Vereinfachtes Modell der Membrantrennung im Wege der organophilen Nanofiltration zeigt Figur 8.

Fig. 8:    Vereinfachtes Modell der organophilen Nanofiltration

Der molare Permeatfluss n"$_{M,i}$ der Komponente i (siehe Figur 8) wird in dem Modell über den Reinkomponentenfluss n"$_{M,i, pure}$ berechnet:

$$n''_{M,i,pure} = P^0_{i,p} \cdot \exp\left(-\alpha_{i,p}\Delta p\right)\Delta p \frac{\eta_{i,pure}(T_{Ref})}{\eta_{i,pure}(T)M_i} \qquad (8)$$

Hier ist Pi,p0 die Standardpermeanz (Massenbezogen) der Membran für die Komponente i bei einem Transmembrandruck von 0 bar. Der Parameter $\alpha_{i,p}$ beschreibt die Kompaktierung der Membran, $\Delta p$ ist der Transmembrandruck und n$_{i,pure}$ die Viskosität des Reinstoffes und Mi das Molargewicht. Mit dem Reinkomponentenfluss und dem molaren Volumen Vi wird die Permeanz der Membran berechnet.

$$P_{i,p} = n''_{M,i,pure} \left/ 1 - \left(\exp\left(\frac{-\Delta p \tilde{V}_i}{RT}\right)\right)\right. \qquad (9)$$

Mit der Permeanz kann schließlich der Permeatfluss n"$_{M,i}$ ermittelt werden:

$$n''_{M,i} = P_{i,p} \cdot \left(x_{R,i} - x_{P,i}\exp\left(\frac{-\Delta p \tilde{V}_i}{RT}\right)\right) \qquad (10)$$

Tabelle 2 zeigt die Standardpermeanzen für die Simulation:

Tabelle 2: Standardpermeanzen

| Stoff | Permeanz ONF 2 |
|---|---|
| Dibuten | 0,074 kg h$^{-1}$ m$^{-2}$ |
| Nonanal | 0,140 kg h$^{-1}$ m$^{-2}$ |
| Nonanol | 0,150 kg h$^{-1}$ m$^{-2}$ |
| Hochsieder | 0,105 kg h$^{-1}$ m$^{-2}$ |
| Komplex | 0,026 kg h$^{-1}$ m$^{-2}$ |
| Ligand | 0,099 kg h$^{-1}$ m$^{-2}$ |

Es gibt verschiedene Einflussfaktoren auf die Wirtschaftlichkeit des Verfahrens. Zum einen sollte der Rhodiumeinsatzfaktor (entspricht dem Verlust von Rh) möglichst niedrig sein, zum anderen sollten die Investitionskosten, die unter anderem von der benötigten Membranfläche abhängen, nicht zu hoch sein.

Bei den betrachteten Varianten beträgt das Gesamtvolumen des Reaktors beträgt 67 m$^3$. Aspekte wie Wärmeübergang oder geometrische Gestaltung des Reaktors wurden bei der Modellierung nicht berücksichtigt. Der Zulauf an Dibuten beträgt 20 t/hr, so dass bei 8500 Betriebsstunden im Jahr eine Ausbeute von 93% zum Produkt Nonanal benötigt wird, um den Weltproduktionsmaßstab von 200 kt/a zu erreichen. Bei einem Restgehalt von 8% Dibuten kann diese Ausbeute nicht erreicht werden, so dass in diesem Fall eine Rückführung der nicht reagierten Dibutene erforderlich ist. Dies ist in den Simulationsrechnungen zu den Verschaltungsvarianten A bis D berücksichtigt. In allen Membranstufen wurden die oNF2 von GMT Membrantechnik GmbH eingesetzt.

Ein Einsatz von anderen Membranen in der gesamten Anlage oder auch nur in Anlagenteilen könnte die Wirtschaftlichkeit weiter verbessern.

Die Membrantemperatur liegt in den Simulationsrechnungen zu den Verschaltungsvarianten A bis D bei 33°C. Durch höhere Betriebstemperaturen der Nanofiltration reduziert sich die Membranfläche, gleichzeitig nimmt der Membranrückhalt für das Katalysatorsystem mit der Zeit schneller ab. Abhängig von den Membranwechselkosten können höhere Betriebstemperaturen wirtschaftlicher sein, um die verbaute Gesamtmembranfläche zu reduzieren. Die transmembrane Druckdifferenz für die durchgeführten Simulationen beträgt 35 bar (3.5 MPa).

Im Folgenden werden vier Ausführungsvarianten A bis D der Erfindung näher untersucht:

Verschaltungsvariante A

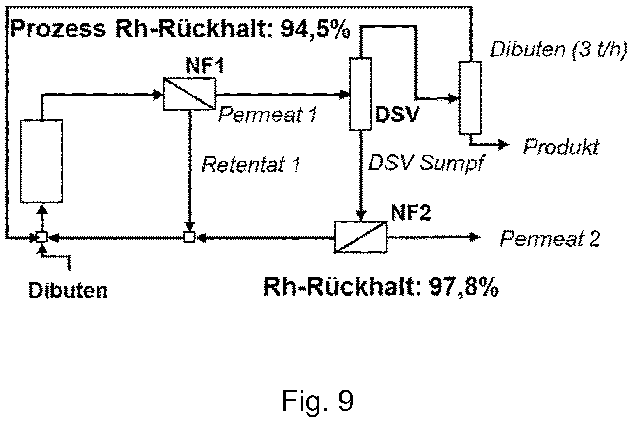

Wegen der hohen Rhodiumverluste in dem Nonanal-Produktstrom bei einer rein membranbasierten Abtrennung, werden in den folgenden Betrachtungen der Hochsieder und das verbliebene Rhodium durch eine thermische Trenneinheit in Gestalt eines Dünnschichtverdampfers (DSV) vom Nonanalstrom hinter der ersten Membrantrennstufe (NF1) abgetrennt. Dies ist in Figur 9 dargestellt.

Figur 9: Verschaltungsvariante A

Durch die geringe Rhodiumkonzentration im Permeat führt die thermische Abtrennung zu einem geringen Verlust an Rhodium durch Verclusterung. Dieser Rhodiumverlust wird in der Simulation nicht berücksichtigt.
Durch die thermische Abtrennung reichern sich die Hochsieder im Katalysatorkreislauf an. Ein Teil des Hochsiederstroms wird deshalb über eine zweite Membrantrenneinheit (NF2) abgetrennt, um eine zu hohe Aufkonzentrierung an Hochsieder im
Katalysatorkreislauf zu vermeiden. Die weitere Aufbereitung der Hochsiederausschleusung, die wegen der noch großen Nonanalkonzentration interessant ist, wird in der Simulation nicht weiter betrachtet. Nach der Abtrennung des Hochsieders wird das nicht reagierte Dibuten, das im Produktstrom noch einen Anteil von ~7% hat, abgetrennt und in den Reaktor zurückgeführt, um das Dibuten vollständig umzusetzen und die geforderte Nonanalproduktion von 200 kta zu erreichen. Die thermische Aufarbeitung des Produktgemisches ist in der Simulation als einfacher Splitter mit festen Splitfaktoren modelliert. Der erste Trennschritt ist ein Flashverdampfer, der bei 40 mbar betrieben wird. Die Temperatur des Verdampfers wird dabei so eingestellt, dass 98% des Hochsieders im Sumpfprodukt bleiben.
Tabelle 3 zeigt die Ergebnisse der Simulation. Die berechneten Membranflächen liegen für die erste Membrantrenneinheit bei 2416 m$^2$ und bei der zweiten Membrantrenneinheit bei 384 m$^2$. Der Rhodiumeinsatzfaktor liegt bei 0,145 g Rhodium pro Tonne Nonanal. Davon werden 38,9% über das Permeat der zweiten Membrantrenneinheit abgeführt. Dieser Anteil kann durch einen Adsorber oder eine weitere Nanofiltrationsstufe auf einfache Weise weiter reduziert werden. Die übrigen 61,1 % sind Verluste durch Verclusterung und Ablagerung in der Anlage. Der Sumpf der thermischen Trenneinheit DSV hat in dieser Verschaltung eine geringere Rhodiumkonzentration als das Retentat der ersten Membrantrenneinheit. Der Anteil der Verclusterung ist vergleichsweise hoch.

Tabelle 3: Ergebnisse der Verschaltungsvariante A

|  | Permeat1 | Retentat1 | Sumpf | Permeat2 |
|---|---|---|---|---|
| Menge [t/h] | 30,1 | 1,2 | 2,8 | 0,4 |
| Dibuten [m-%] | 10,5% | 6,4% | 1,1% | 0,8% |
| Nonanal[m-%] | 82,7% | 47,3% | 45,5% | 51,4% |
| Nonanol[m-%] | 2,1% | 1,3% | 3,9% | 4,4% |
| Hochsieder [m-%] | 4,7% | 45,0% | 49,5% | 43,4% |
| Rhodium [ppm] | 12,2 | 222,1 | 130,9 | 3,3 |

Verschaltungsvariante B

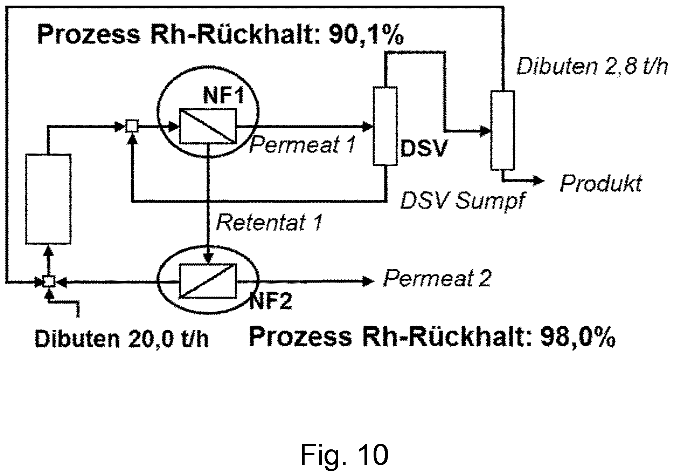

In Variante B wird der Sumpf der thermischen Trenneinheit (DSV) vor die erste einstufige Membrantrenneinheit (NF1) geführt. Die Ausschleusung der Hochsieder erfolgt über die Zufuhr des Retentats der ersten Membrantrenneinheit (NF1) auf die zweite zweistufige Membrantrenneinheit (Verstärkungskaskade NF2). Diese Verschaltung ist in Figur 10 dargestellt.

Figur 10: Verschaltungsvariante B

Tabelle 4 zeigt die Ergebnisse der Simulation. Die berechneten Membranflächen liegen für die erste Membrantrenneinheit bei 3032 m$^2$ und bei der zweiten Membrantrenneinheit bei 392 m$^2$. Der Rhodiumeinsatzfaktor liegt bei 0,144 g Rhodium pro Tonne Nonanal. Davon werden 40,3% über das Permeat der zweiten Nanofiltration abgeführt. Dieser Anteil kann durch einen Scavenger oder eine weitere Nanofiltrationsstufe auf einfache Weise weiter reduziert werden. Die übrigen 59,7% sind Verluste durch Verclusterung und Ablagerung in der Anlage.

Tabelle 4: Ergebnisse der Verschaltungsvariante B

|  | Permeat1 | Retentat1 | Sumpf | Permeat2 |
|---|---|---|---|---|
| Menge [t/h] | 30,9 | 4 | 3,7 | 0,4 |

(fortgesetzt)

| | Permeat1 | Retentat1 | Sumpf | Permeat2 |
|---|---|---|---|---|
| Dibuten [m-%] | 9,7% | 6,4% | 1,0% | 6,3% |
| Nonanal[m-%] | 80,8% | 50,5% | 36,2% | 56,9% |
| Nonanol[m-%] | 2,2% | 1,4% | 3,3% | 1,6% |
| Hochsieder [m-%] | 7,3% | 41,6% | 59,5% | 35,3% |
| Rhodium [ppm] | 15,5 | 157 | 129,4 | 3,5 |

Verschaltungsvariante C

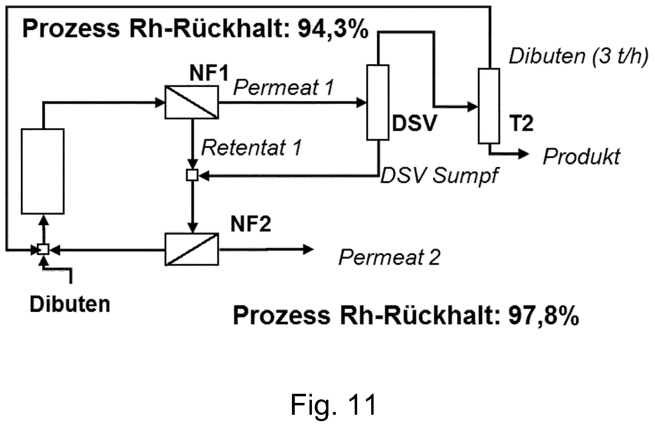

In Figur 11 ist die Verschaltungsvariante C dargestellt. Hier werden das Retentat der ersten Membrantrenneinheit (NF1) und der Sumpf der thermischen Trenneinheit (DSV) vor der zweiten Membrantrenneinheit (NF2) vermischt und dann zur Hochsiederausschleusung in die zweite Membrantrenneinheit gefahren. Damit weiterhin ein Katalysatorkreislauf von 4t/h eingehalten werden kann, wurde die Retentatmenge der ersten Membrantrenneinheit auf 1,2t/h reduziert. Das Retentat der zweiten Membrantrennstufe wird vor den Reaktor rezykliert.

Figur 11:    Verschaltungsvariante C

Tabelle 5 zeigt die Ergebnisse der Simulation. Die berechneten Membranflächen liegen für die erste Nanofiltration bei 2473 m$^2$ und bei der zweiten Nanofiltration bei 388 m$^2$. Der Rhodiumeinsatzfaktor liegt bei 0,152 g Rhodium pro Tonne Nonanal. Davon werden 42,1% über das Permeat der zweiten Nanofiltration abgeführt. Dieser Anteil kann durch einen Scavenger oder eine weitere Nanofiltrationsstufe auf einfache Weise weiter reduziert werden. Die übrigen 57,9% sind Verluste durch Verclusterung und Ablagerung in der Anlage.

Tabelle 5: Ergebnisse der Verschaltungsvariante C

| | Permeat1 | Retentat1 | Sumpf | Permeat2 |
|---|---|---|---|---|
| Menge [t/h] | 30,2 | 1,2 | 2,9 | 0,4 |
| Dibuten [m-%] | 10,5% | 6,4% | 1,1% | 2,4% |
| Nonanal[m-%] | 82,4% | 46,6% | 44,3% | 51,1% |
| Nonanol[m-%] | 2,1% | 1,2% | 3,8% | 3,5% |
| Hochsieder [m-%] | 5,0% | 45,8% | 50,8% | 43,0% |
| Rhodium [ppm] | 12,4 | 217,8 | 129,6 | 3,8 |

Verschaltungsvariante D

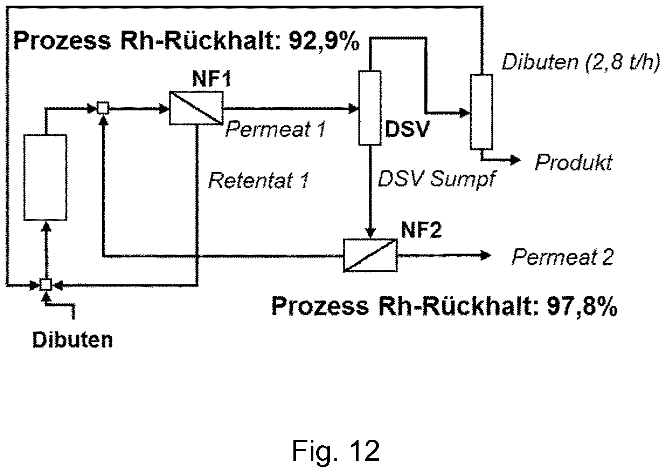

Die in Figur 12 dargestellte Verschaltungsvariante D zeigt eine Ausführung, bei der wie in Variante A der Sumpf der thermischen Trenneinrichtung (DSV) auf die zweite Membrantrenneinheit (NF2) zwecks Hochsiederausschleusung gefahren wird. Das Retentat der zweiten Membrantrenneinheit wird jedoch mit dem Hydroformylierungsaustrag vermischt und der ersten Membrantrenneinheit zugeführt und nicht wie bei Variante C vor den Reaktor zurückgeführt.

Figur 12:    Verschaltungsvariante D

Tabelle 6 zeigt die Ergebnisse der Simulation. Die berechneten Membranflächen liegen für die erste Membrantrenneinheit (NF1) bei 2324 m$^2$ und bei der zweiten Membrantrenneinheit (NF2) bei 382 m$^2$. Der Rhodiumeinsatzfaktor liegt bei 0,138 g Rhodium pro Tonne Nonanal. Davon werden 37,7% über das Permeat der zweiten Membrantrenneinheit abgeführt. Dieser Anteil kann durch einen Adsorber oder eine weitere Nanofiltrationsstufe auf einfache Weise weiter reduziert werden.

Die übrigen 62,3% sind Verluste durch Verclusterung und Ablagerung in der Anlage. Im Sumpf der thermischen Trenneinheit ist die Konzentration geringer als im Retentat der ersten Membrantrenneinheit, was zu einer verminderten Verclusterung führt. Zudem kann ein größerer Retentatstrom bei der ersten Membrantrenneinheit gefahren werden als bei den Verschaltungen A und C.

Tabelle 6 : Ergebnisse der Verschaltungsvariante D

| | Permeat1 | Retentat1 | Sumpf | Permeat2 |
|---|---|---|---|---|
| Menge [t/h] | 29,9 | 4 | 2,7 | 0,4 |

(fortgesetzt)

|  | Permeat1 | Retentat1 | Sumpf | Permeat2 |
|---|---|---|---|---|
| Dibuten [m-%] | 10,1% | 7,3% | 1,1% | 0,8% |
| Nonanal [m-%] | 83,3% | 57,7% | 47,4% | 53,3% |
| Nonanol [m-%] | 2,2% | 1,6% | 4,1% | 4,7% |
| Hochsieder [m-%] | 4,3% | 33,4% | 47,4% | 41,2% |
| Rhodium [ppm] | 11,3 | 159,3 | 125,4 | 3,1 |

Fazit

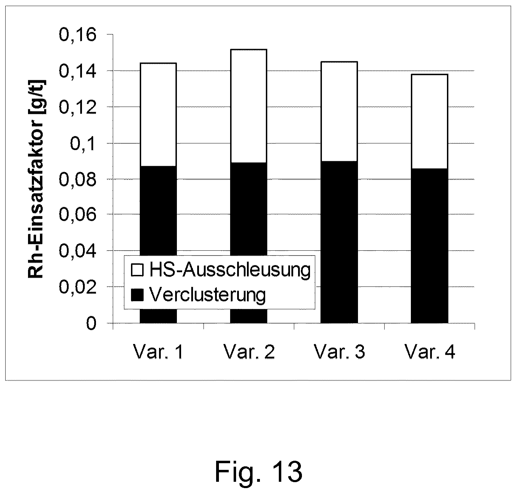

In den Figuren 13 bis 16 sind die Ergebnisse der simulierten Verschaltungsvarianten A bis D graphisch gegenüber gestellt.

Fig. 13: Vergleich der Ergebnisse der Simulationsrechnungen hinsichtlich Rh-Einsatzfaktor;

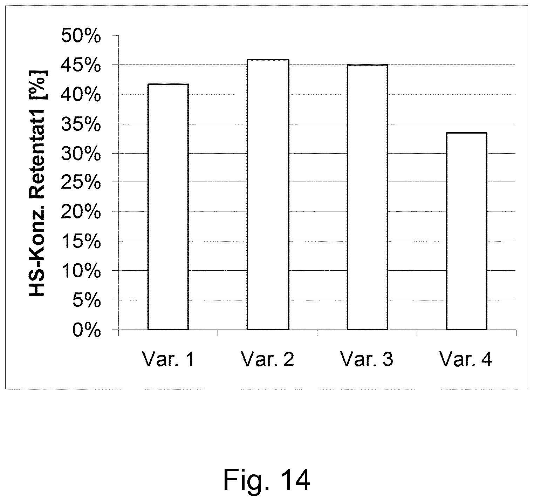

Fig. 14: Vergleich der Ergebnisse der Simulationsrechnungen hinsichtlich der Hochsiederkonzentration im Retentat;

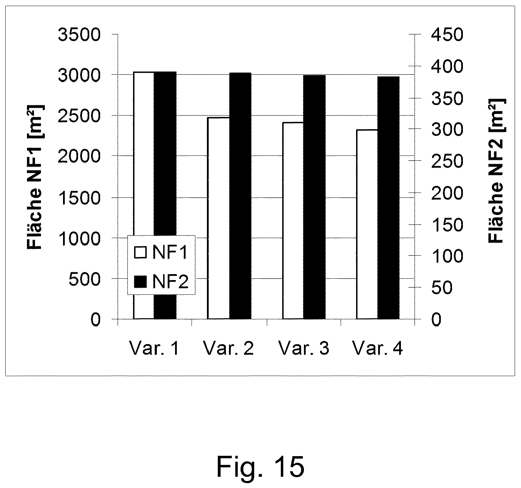

Fig. 15: Vergleich der Ergebnisse der Simulationsrechnungen hinsichtlich des Membranflächenbedarfs;

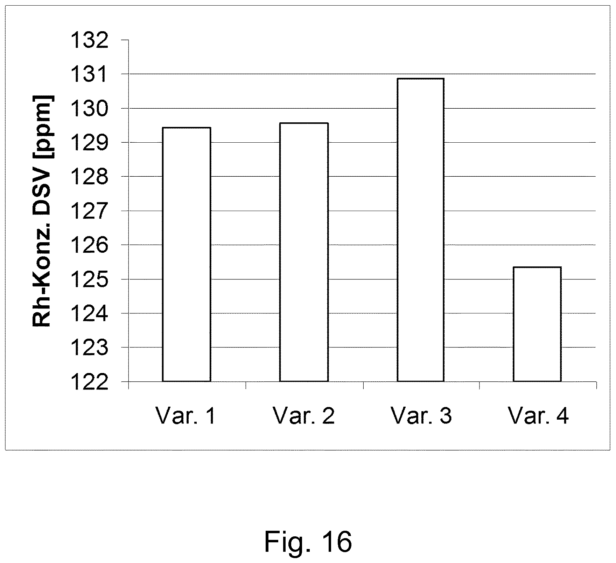

Fig. 16: Vergleich der Ergebnisse der Simulationsrechnungen hinsichtlich der Rhodiumkonzentration in der Trenneinheit.

Der Vergleich der graphischen Darstellung in den Figuren 13 bis 16 zeigt, dass Variante D hinsichtlich aller für die Wirtschaftlichkeit der Produktabtrennung relevanten Parameter am günstigsten ist. Variante D lässt die geringsten Rhodiumverluste durch Ausschleusung und Verclusterung zu und weist zudem den geringsten Membranflächenbedarf auf.

Von allen erfindungsgemäßen Ausführungsformen A bis D ist die durch die Rückführung des Retentats der zweiten Membrantrennstufe vor die erste Membrantrennstufe gekennzeichnete Verschaltungsvariante D deswegen die bevorzugte.

**Bezugszeichenliste**

| | |
|---|---|
| 1 | Hydroformylierungsreaktor |
| 2 | Olefin |
| 3 | Synthesegas |
| 4 | Hydroformylierungsaustrag |
| 5 | erster Wärmetauscher |
| 6 | erste Membrantrenneinheit |
| 7 | Produktstrom |
| 8 | Reaktorrücklauf / primäres Rezyklat |
| 9 | Entgaser |
| 10 | thermische Trenneinheit |
| 11 | Kopfprodukt |
| 12 | Sumpfprodukt |
| 13 | erster Adsorber |
| 14 | Hydrierung |
| 15 | Hydrierungsgemisch |
| 16 | thermische Aufarbeitung |
| 17 | Alkoholreiche Fraktion |
| 18 | Leichtsiederfraktion |
| 19 | Hochsiederfraktion |
| 20 | zweite Membrantrenneinheit |
| 21 | Permeat |
| 22 | Retentat / sekundäres Rezyklat |

23    zweiter Wärmetauscher
24    zweiter Adsorber
25    gemeinsamer Adsorber
26    erste Destillationskolonne
27    zweite Destillationskolonne
28    dritte Destillationskolonne
29    Sumpf erste Destillationskolonne
30    Sumpf zweite Destillationskolonne
31    Fallfilmverdampfer
32    Dünnschichtverdampfer
33    Fallrohre
34    Heizmantel
35    Dampfeinlass
36    Dampfauslass
37    Gasaustritt des Fallfilmverdampfers
38    Sumpf des Fallfilmverdampfers
39    Tubus
40    Rotor
41    Wischorgane
42    Gasaustritt des Dünnschichtverdampfers
43    Sumpf des Dünnschichtverdampfers
44    Zweiter Fallfilmverdampfer
45    Sumpf des zweiten Fallfilmverdampfers
46    Pumpe der Abtriebskaskade
47    erste Stufe der Abtriebskaskade
48    Retentat der ersten Stufe der Abtriebskaskade
49    zweite Stufe der Abtriebskaskade
50    Permeat der zweiten Stufe der Abtriebskaskade / Permeatrückführung
51    erste Druckerhöhungspumpe der Verstärkungskaskade
52    erste Stufe der Verstärkungskaskade
53    Permeat der ersten Stufe der Verstärkungskaskade
54    zweite Druckerhöhungspumpe der Verstärkungskaskade
55    zweite Stufe der Verstärkungskaskade
56    Retentat der zweiten Stufe der Verstärkungskaskade / Retentatrückführung

**Patentansprüche**

1.  Verfahren zur Herstellung von Alkoholen durch homogen katalysierte Hydroformylierung von Olefinen zu Aldehyden und anschließender Hydrierung der Aldehyde mit den folgenden Schritten:

    a) Bereitstellen von mindestens einem Olefin, von Synthesegas und von einem Katalysatorsystem, sowie optional von einem Lösungsmittel;
    b) Hydroformylieren des Olefins in Gegenwart des Synthesegases und des Katalysatorsystems in mindestens einem Hydroformylierungsreaktor unter Bildung von zumindest Aldehyd sowie von Hochsiedern;
    c) Abziehen eines flüssigen Hydroformylierungsaustrags umfassend Aldehyd, Olefin, gelöstes Synthesegas, Katalysatorsystem und Hochsieder aus dem Hydroformylierungsreaktor;
    d) optionales Entgasen des flüssigen Hydroformylierungsaustrags;
    e) Auftrennen des flüssigen Hydroformylierungsaustrags in einer ersten Membrantrenneinheit in einen Produktstrom und in einen Reaktorrücklauf, wobei das Katalysatorsystem im Reaktorrücklauf angereichert wird;
    f) Rückführen des Reaktorrücklaufs in den Hydroformylierungsreaktor;
    g) optionales Entgasen des Produktstroms;
    h) Auftrennen des Produktstroms in einer thermischen Trenneinheit in ein gasförmiges Kopfprodukt umfassend Aldehyd und Olefin und in ein flüssiges Sumpfprodukt umfassend Aldehyd, Hochsieder und Katalysatorkomplex;
    i) Auftrennen des Sumpfprodukts in einer zweiten Membrantrenneinheit in ein Permeat und in ein Retentat, wobei das Katalysatorsystem im Retentat angereichert wird;

    wobei die Verbesserung darin besteht, dass

k) die thermische Trenneinheit dergestalt betrieben wird, dass 80% bis 98% der mit dem Produktstrom in die thermische Trenneinheit eingebrachten Masse als Kopfprodukt die thermische Trenneinheit wieder verlässt;
l) und dass sowohl zumindest ein Teil des Kopfprodukts der thermischen Trenneinheit, als auch das Permeat der zweiten Membrantrenneinheit einer gemeinsamen oder getrennten Hydrierung zugeführt werden.

2. Verfahren nach Anspruch 1,
**dadurch gekennzeichnet,**
**dass** das Retentat der zweiten Membrantrenneinheit mit dem aus dem Hydroformylierungsreaktor abgezogenen, flüssigen Hydroformylierungsaustrag vermischt der ersten Membrantrenneinheit zugeführt wird.

3. Verfahren nach Anspruch 1 oder 2,
**dadurch gekennzeichnet,**
**dass** das Permeat der zweiten Membrantrenneinheit vor der Hydrierung durch einen Adsorber gefahren wird.

4. Verfahren nach einem der Ansprüche 1 bis 3,
**dadurch gekennzeichnet,**
**dass** das Kopfprodukt der thermischen Trenneinheit vor der Hydrierung durch einen Adsorber gefahren wird.

5. Verfahren nach Anspruch 3 und 4,
**dadurch gekennzeichnet,**
**dass** das Kopfprodukt der thermischen Trenneinheit und das Permeat der zweiten Membrantrenneinheit durch denselben Adsorber gefahren werden.

6. Verfahren nach Anspruch 5,
**dadurch gekennzeichnet,**
**dass** das Kopfprodukt der thermischen Trenneinheit und das Permeat der zweiten Membrantrenneinheit einer gemeinsamen Hydrierung zugeführt werden.

7. Verfahren nach einem der Ansprüche 1 bis 6,
**dadurch gekennzeichnet,**
**dass** aus der Hydrierung ein Hydrierungsgemisch abgezogen und unter Erhalt einer Alkoholreichen Fraktion, einer Leichtsiederfraktion und einer Hochsiederfraktion einer thermischen Aufarbeitung unterzogen wird.

8. Verfahren nach einem der Ansprüche 1 bis 7,
**dadurch gekennzeichnet,**
**dass** die thermische Trenneinheit einen Dünnschichtverdampfer und einen Fallfilmverdampfer umfasst, wobei Dünnschichtverdampfer und Fallfilmverdampfer seriell miteinander verschaltet sind, insbesondere, dass der Dünnschichtverdampfer seriell hinter den Fallfilmverdampfer geschaltet ist.

9. Verfahren nach einem der Ansprüche 1 bis 7,
**dadurch gekennzeichnet,**
**dass** die thermische Trenneinheit zwei oder drei seriell miteinander verschaltete Fallfilmverdampfer umfasst.

10. Verfahren nach einem der Ansprüche 1 bis 9,
**dadurch gekennzeichnet,**
**dass** die erste Membrantrenneinheit als eine zweistufige Abtriebskaskade ausgeführt ist, insbesondere, dass dabei die genutzte Membranfläche der Konzentratloops kleiner ist als die genutzte Membranfläche der übrigen Loops.

11. Verfahren nach einem der Ansprüche 1 bis 10,
**dadurch gekennzeichnet,**
**dass** die zweite Membrantrenneinheit als eine zweistufige Verstärkungskaskade ausgeführt ist.

12. Verfahren nach einem der Ansprüche 1 bis 11,
**dadurch gekennzeichnet,**
**dass** es sich bei dem Katalysatorsystem um einen Rhodiumkatalysator mit einem Organo-Phosphor-Liganden handelt, wobei der Ligand ausgewählt ist aus Phosphiten, Phosphinen oder Phosphoramiditen, wobei Phosphit-Liganden besonders bevorzugt gewählt sind.

**13.** Verfahren nach einem der Ansprüche 1 bis 12,
**dadurch gekennzeichnet,**
**dass** Olefine mit acht Kohlenstoffatomen zu Aldehyden mit neun Kohlenstoffatomen hydroformyliert und die Aldehyde zu Alkohol mit neun Kohlenstoffatomen hydriert werden.

## Claims

**1.** Process for producing alcohols by homogeneously catalysed hydroformylation of olefins to aldehydes and subsequent hydrogenation thereof, comprising the steps of:

a) providing one or more olefins, syngas and a catalyst system and also optionally a solvent;
b) hydroformylating the olefin(s) in the presence of the syngas and of the catalyst system in one or more than one hydroformylation reactor to form at least aldehyde and also high boilers;
c) withdrawing a liquid hydroformylation effluent comprising aldehyde, olefin, dissolved syngas, catalyst system and high boilers from the hydroformylation reactor;
d) optionally devolatilizing the liquid hydroformylation effluent;
e) separating the liquid hydroformylation effluent in a first membrane separation unit into a product stream and a reactor return stream, wherein the catalyst system partitions into the reactor return stream;
f) returning the reactor return stream into the hydroformylation reactor;
g) optionally devolatilizing the product stream;
h) separating the product stream in a thermal separation unit into a gaseous head product comprising aldehyde and olefin and a liquid bottom product comprising aldehyde, high boilers and catalyst complex;
i) separating the bottom product in a second membrane separation unit into a permeate and a retentate, wherein the catalyst system partitions into the retentate;

wherein the improvement consists in

k) operating the thermal separation unit such that 80% to 98% of the mass introduced into the thermal separation unit with the product stream re-emerges from the thermal separation unit as head product;
1) and subjecting at least some of the head product of the thermal separation unit and the permeate of the second membrane separation unit to conjoint or separate hydrogenation.

**2.** Process according to Claim 1,
**characterized in that**
the retentate of the second membrane separation unit is fed to the first membrane separation unit in admixture with the liquid hydroformylation effluent withdrawn from the hydroformylation reactor.

**3.** Process according to Claim 1 or 2,
**characterized in that**
the permeate of the second membrane separation unit passes through an adsorber before hydrogenation.

**4.** Process according to any of Claims 1 to 3,
**characterized in that**
the head product of the thermal separation unit passes through an adsorber before hydrogenation.

**5.** Process according to Claims 3 and 4,
**characterized in that**
the head product of the thermal separation unit and the permeate of the second membrane separation unit pass through the same adsorber.

**6.** Process according to Claim 5,
**characterized in that**
the head product of the thermal separation unit and the permeate of the second membrane separation unit are subject to conjoint hydrogenation.

**7.** Process according to any of Claims 1 to 6,
**characterized in that**

a hydrogenation mixture is withdrawn from the hydrogenation and subjected to a thermal work-up to obtain an alcohol-rich fraction, a low-boiler fraction and a high-boiler fraction.

8. Process according to any of Claims 1 to 7,
   **characterized in that**
   the thermal separation unit comprises a thin film evaporator and a falling film evaporator, wherein the thin film evaporator and the falling film evaporator are serially interconnected, particularly with the thin film evaporator being serially connected downstream of the falling film evaporator.

9. Process according to any of Claims 1 to 7,
   **characterized in that**
   the thermal separation unit comprises two or three serially interconnected falling film evaporators.

10. Process according to any of Claims 1 to 9,
    **characterized in that**
    the first membrane separation unit takes the form of a two-stage depleting cascade, particularly wherein the membrane area used in the concentrate loops is smaller than the membrane area used in the other loops.

11. Process according to any of Claims 1 to 10,
    **characterized in that**
    the second membrane separation unit takes the form of a two-stage enriching cascade.

12. Process according to any of Claims 1 to 11,
    **characterized in that**
    the catalyst system comprises a rhodium catalyst having an organophosphorus ligand, wherein the ligand is selected from phosphites, phosphines or phosphoramidites, wherein the choice of phosphite ligands is particularly preferable.

13. Process according to any of Claims 1 to 12,
    **characterized in that**
    olefins having eight carbon atoms are hydroformylated to aldehydes having nine carbon atoms and the aldehydes are hydrogenated to alcohol having nine carbon atoms.

**Revendications**

1. Procédé de fabrication d'alcools par hydroformylation sous catalyse homogène d'oléfines en aldéhydes, puis hydrogénation des aldéhydes selon les étapes suivantes :

   a) la préparation d'au moins une oléfine, d'un gaz de synthèse et d'un système catalytique, ainsi qu'éventuellement d'un solvant ;
   b) l'hydroformylation de l'oléfine en présence du gaz de synthèse et du système catalytique dans au moins un réacteur d'hydroformylation avec formation d'au moins un aldéhyde et de composants de point d'ébullition élevé ;
   c) le soutirage d'une sortie d'hydroformylation liquide comprenant l'aldéhyde, l'oléfine, le gaz de synthèse dissous, le système catalytique et les composants de point d'ébullition élevé du réacteur d'hydroformylation ;
   d) éventuellement le dégazage de la sortie d'hydroformylation liquide ;
   e) la séparation de la sortie d'hydroformylation liquide dans une première unité de séparation sur membrane en un courant de produits et en un reflux de réacteur, le système catalytique s'accumulant dans le reflux de réacteur ;
   f) le recyclage du reflux de réacteur dans le réacteur d'hydroformylation ;
   g) éventuellement le dégazage du courant de produits ;
   h) la séparation du courant de produits dans une unité de séparation thermique en un produit de tête gazeux comprenant l'aldéhyde et l'oléfine et en un produit de fond liquide comprenant l'aldéhyde, les composants de point d'ébullition élevé et le complexe catalytique ;
   i) la séparation du produit de fond dans une deuxième unité de séparation sur membrane en un perméat et en un rétentat, le système catalytique s'accumulant dans le rétentat ;

   l'amélioration consistant en ce que

k) l'unité de séparation thermique est exploitée de sorte que 80 % à 98 % de la masse introduite avec le courant de produits dans l'unité de séparation thermique quitte l'unité de séparation thermique en tant que produit de tête ;
1) et en ce qu'aussi bien au moins une partie du produit de tête de l'unité de séparation thermique que le perméat de la deuxième unité de séparation sur membrane sont introduits dans une hydrogénation commune ou séparée.

2. Procédé selon la revendication 1, **caractérisé en ce que** le rétentat de la deuxième unité de séparation sur membrane est introduit mélangé avec la sortie d'hydroformylation liquide soutirée du réacteur d'hydroformylation dans la première unité de séparation sur membrane.

3. Procédé selon la revendication 1 ou 2, **caractérisé en ce que** le perméat de la deuxième unité de séparation sur membrane est conduit au travers d'un adsorbeur avant l'hydrogénation.

4. Procédé selon l'une quelconque des revendications 1 à 3, **caractérisé en ce que** le produit de tête de l'unité de séparation thermique est conduit au travers d'un adsorbeur avant l'hydrogénation.

5. Procédé selon les revendications 3 et 4, **caractérisé en ce que** le produit de tête de l'unité de séparation thermique et le perméat de la deuxième unité de séparation sur membrane sont conduits au travers du même adsorbeur.

6. Procédé selon la revendication 5, **caractérisé en ce que** le produit de tête de l'unité de séparation thermique et le perméat de la deuxième unité de séparation sur membrane sont introduits dans une hydrogénation commune.

7. Procédé selon l'une quelconque des revendications 1 à 6, **caractérisé en ce qu'**un mélange d'hydrogénation est soutiré de l'hydrogénation et soumis à un traitement thermique avec obtention d'une fraction riche en alcool, d'une fraction de composants de point d'ébullition faible et d'une fraction de composants de point d'ébullition élevé.

8. Procédé selon l'une quelconque des revendications 1 à 7, **caractérisé en ce que** l'unité de séparation thermique comprend un évaporateur à couche mince et un évaporateur à film tombant, l'évaporateur à couche mince et l'évaporateur à film tombant étant raccordés l'un avec l'autre en série, notamment **en ce que** l'évaporateur à couche mince est raccordé en série après l'évaporateur à film tombant.

9. Procédé selon l'une quelconque des revendications 1 à 7, **caractérisé en ce que** l'unité de séparation thermique comprend deux ou trois évaporateurs à film tombant raccordés les uns avec les autres en série.

10. Procédé selon l'une quelconque des revendications 1 à 9, **caractérisé en ce que** la première unité de séparation sur membrane est configurée sous la forme d'une cascade de rectification à deux niveaux, notamment **en ce que** la surface de membrane utilisée des boucles de concentré est inférieure à la surface de membrane utilisée des autres boucles.

11. Procédé selon l'une quelconque des revendications 1 à 10, **caractérisé en ce que** la deuxième unité de séparation sur membrane est configurée sous la forme d'une cascade d'enrichissement à deux niveaux.

12. Procédé selon l'une quelconque des revendications 1 à 11, **caractérisé en ce que** le système catalytique consiste en un catalyseur de rhodium comprenant un ligand organophosphore, le ligand étant choisi parmi les phosphites, les phosphines ou les phosphoramidites, les ligands phosphite étant choisis de manière particulièrement préférée.

13. Procédé selon l'une quelconque des revendications 1 à 12, **caractérisé en ce que** des oléfines contenant huit atomes de carbone sont hydroformylées en aldéhydes contenant neuf atomes de carbone et les aldéhydes sont hydrogénés en alcools contenant neufs atomes de carbone.

Fig. 1

EP 2 961 529 B1

Fig. 2

EP 2 961 529 B1

Fig. 3

Fig. 4

Fig. 5

Fig. 6

EP 2 961 529 B1

Fig. 7

Permeat

Retentat

$$x_{P,i}$$
$$n''_{M,i}\left(\Delta p, \widetilde{V}_i, T, \eta_i\right)$$
$$x_{R,i}$$

Feed

Fig. 8

Fig. 9

EP 2 961 529 B1

Fig. 10

EP 2 961 529 B1

EP 2 961 529 B1

**Prozess Rh-Rückhalt: 94,3%**

NF1 · *Permeat 1*

DSV

*Dibuten (3 t/h)*

T2

*Retentat 1*

→ *Produkt*

*DSV Sumpf*

NF2

*Permeat 2*

**Dibuten**

**Prozess Rh-Rückhalt: 97,8%**

Fig. 11

Fig. 12

Fig. 13

EP 2 961 529 B1

Fig. 14

Fig. 15

Fig. 16

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102008007080 A1
- EP 2220017 B1
- EP 1674441 B1
- EP 1193239 B1
- EP 1931472 B1
- WO 2009049911 A1
- WO 2006013060 A1
- WO 2010097428 A1
- EP 1603663 B1
- EP 0781166 A
- EP 0987240 B1
- EP 0987241 B1
- EP 2401078 A1

**In der Beschreibung aufgeführte Nicht-Patentliteratur**

- *CHEMICAL ABSTRACTS,* 27458-94-2
- **FALBE, JÜRGEN.** New Syntheses with Carbon Monoxide. Springer, 1980
- **PRUETT, ROY L.** Hydroformylation. *Advances in Organometallic Chemistry,* 1979, vol. 17, 1-60
- Hydroformylation (Oxo Synthesis, Roelen Reaction). **FROHNING, CARL D. ; KOHLPAINTNER, CHRISTIAN W.** Applied homogeneous catalysis with organometallic compounds. Wiley, 1996, 29-104
- Rhodium Catalyzed Hydroformylation. Catalysis by Metal Complexes. 2000, vol. 22
- Membranverfahren. **MELIN ; RAUTENBACH.** Grundlagen der Modul- und Anlagenauslegung. Springer, 2004
- **PRISKE, M. et al.** Reaction integrated separation of homogeneous catalysts in the hydroformylation of higher olefins by means of organophilic nanofiltration. *Journal of Membrane Science,* 15. September 2010, vol. 360 (1-2), 77-83
- Evaporation. **BILLET ; REINHARD.** Ullmann's Encyclopedia of Industrial Chemistry. 15. Juni 2000
- Bubble Columns. **DEEN, N.G. ; MUDDE, R.F. ; KUIPERS, J.A.M. ; ZEHNER, P. ; KRAUME, M.** Ullmann's Encyclopedia of Industrial Chemistry. 15. Januar 2010
- *CHEMICAL ABSTRACTS,* 10071-47-9
- Membranes. **VON I. CABASSO.** Encyclopedia of Polymer Sience and Technlogy. John Wiley and Sons, 1987